(19)

Europäisches Patentamt
European Patent Office
Office européen des brevets

(11) **EP 4 722 358 A1**

(12) **EUROPEAN PATENT APPLICATION**
published in accordance with Art. 153(4) EPC

(43) Date of publication:
**08.04.2026  Bulletin 2026/15**

(21) Application number: **24815909.7**

(22) Date of filing: **31.05.2024**

(51) International Patent Classification (IPC):
***C12N 9/88*** *(2006.01)*      ***C12P 7/52*** *(2006.01)*

(52) Cooperative Patent Classification (CPC):
**C12N 9/88; C12P 7/52**

(86) International application number:
**PCT/KR2024/007479**

(87) International publication number:
**WO 2024/248535 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **02.06.2023  KR 20230071438
29.05.2024  KR 20240069886**

(71) Applicant: **AJOU UNIVERSITY INDUSTRY-
ACADEMIC
COOPERATION FOUNDATION
Gyeonggi-do 16499 (KR)**

(72) Inventors:
• **YOO, Tae Hyeon
Yongin-si Gyeonggi-do 17003 (KR)**

• **NA, Chae-Yeong
Seoul 08797 (KR)**
• **NASIR, Abdul
Mardan KPK, 23200 (PK)**
• **PARK, Ye Seop
Suwon-si Gyeonggi-do 16501 (KR)**
• **YEON, Young Joo
Gangneung-si Gangwon-do 25514 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) **GLYCEROL DEHYDRATASE VARIANT AND USE THEREOF**

(57)    The present invention relates to a glycerol dehydratase variant and, more specifically, to a fusion glycerol dehydratase variant alleviating the inactivation of coenzyme B12 by oxygen or improving activity for glycerol, which is a substrate, and thus exhibits excellent activity even under conditions of low coenzyme B12 concentration.

[FIG.9]

## Description

## Technical Field

The present invention relates to a glycerol dehydratase variant and the use thereof, and more particularly, to a fused glycerol dehydratase variant that exhibits excellent activity even under conditions of low concentrations of coenzyme B12 by alleviating inactivation of coenzyme B12 by oxygen or improving activity to glycerol as a substrate, and the use thereof.

## Background Art

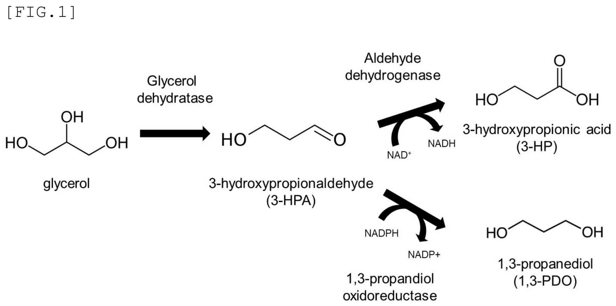

Glycerol dehydratase has an advantage of utilizing glycerol, a byproduct of biodiesel production. Glycerol is converted through glycerol dehydratase to 3-hydroxypropionaldehyde (3-HPA), which is converted to 3-hydroxypropionic acid (3-HP) through aldehyde dehydrogenase, or is converted to 1,3-PDO through 1,3-propanediol oxidoreductase (FIG. 1). Such a simple pathway has led many researchers to study the production of 3-HP and 1,3-PDO via this pathway in various microorganisms. (Drozdzynska, A., et al., BioTechnologia, 92(1), 92-100. (2011); Valdehuesa, et al., Applied microbiology and biotechnology, 97, 3309-3321. (2013); Mitrea, L., et al., Microbial Cell Factories, 16, 1-17. (2017)).

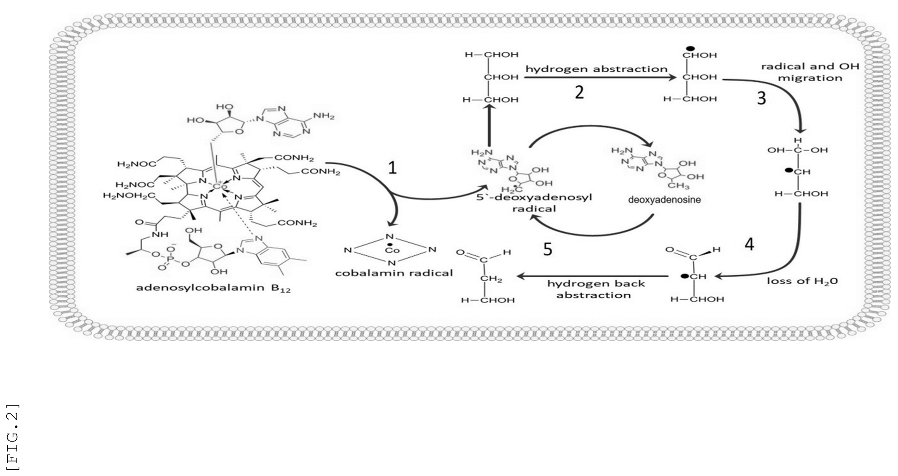

Coenzyme B12-dependent glycerol dehydratases have been widely used in the reaction despite the requirement for expensive vitamin B12. The cobalamin derivative used as a coenzyme in the coenzyme B12-dependent glycerol dehydratase reaction is a very complicated organic cofactor found in nature. Cobalamin (adenosylcobalamin, AdoCbl) which is an active form of coenzyme B12-dependent glycerol dehydratase is located in a pocket formed at the interface of the $\alpha$ and $\beta$ subunits. Cobalamin has a cyclic tetrapyrrole (corrin ring) structure containing a cobalt atom at the center thereof. The corrin ring has four propionamide, three acetamide, and eight methyl groups, as well as a nucleotide tail containing dimethylbenzamidazole (DBI) (FIG. 2). These peripheral groups are well disposed at the $\alpha$, $\beta$ subunit interface for radical reaction-based catalysis and interact with residues at the interface of the enzyme (Toraya, T., et al., Biochemistry 18(3), 417-426. (1979); Toraya, T., & Abeles, R. H. Archives of Biochemistry and Biophysics, 203(1), 174-180. (1980)). The coenzyme B12-dependent glycerol dehydratase shown in FIG. 2 converts glycerol to 3-hydroxypropionaldehyde in accordance with the following mechanism: (1) the binding of the glycerol substrate to glycerol dehydratase induces a structural change in the enzyme, which increases the bond length between the cobalt atom of coenzyme B12 and the adenosyl (red) moiety to 2.5Å from 1.95 to 2.2Å, breaking the carbon-cobalt bond. (2) the adenosyl radical thus produced rotates along the glycosidic bond of the substrate, abstracting a hydrogen atom from the substrate to form a substrate radical. (3) Then, the OH group of the second carbon moves to the terminal carbon to produce a new radical at the second carbon. (4) The produced 1,1-diol is unstable and readily releases water and is converted to an aldehyde group. (5) The hydrogen atom is then abstracted back to 5'deoxyadenosine, to ultimately produce an aldehyde product and an adenosyl radical (Mancia, et al., Structure, 4(3), 339-350. (1996); Shibata, N., et al., Structure, 7(8), 997-1008. (1999); Kamachi, T., et al., Chemistry-A European Journal, 13(28), 7864-7873. (2007)).

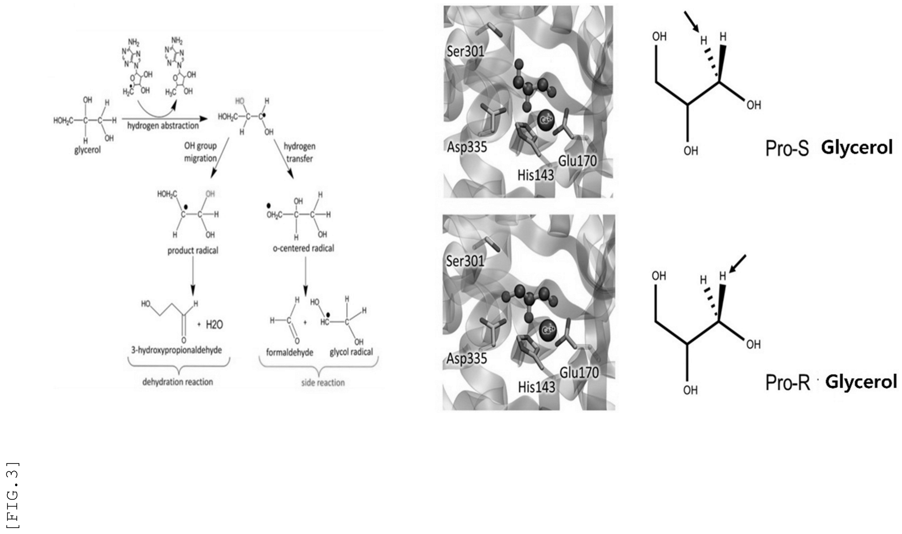

However, the glycerol dehydratase is disadvantageously inactivated when coenzyme B12, which has been deactivated by the substrate glycerol or oxygen, binds to the active site of the enzyme. In this regard, glycerol acts as both a substrate and an inhibitor of glycerol dehydratase. Nevertheless, glycerol dehydratase can convert substrates without inhibition for a short period of time. The cause of the inactivation is considered to be the chirality of the substrate. Although glycerol is not a chiral molecule, it is classified as a pro-chiral molecule in which a single modification of the molecule confers chirality. Studies on diol dehydratase, which is known to have a mechanism very similar to glycerol dehydratase (Poznanskaja, A. et al., Archives of Biochemistry and Biophysics, 194(2), 379-386. (1979); Yamanishi, M., et al., European journal of biochemistry, 269(18), 4484-4494. (2002)), have revealed that there are two binding modes of glycerol, pro-R and pro-S forms, depending on the position of the abstracted hydrogen, and when the substrate is bound in the pro-R form, the dehydration reaction is more dominant, whereas when the substrate is bound in the pro-S form, the inactivation reaction is more dominant (Doitomi, K., et al., Bulletin of the Chemical Society of Japan, 87(9), 950-959. (2014)). The $C_3$-OH group of the pro-S form faces serine at position 301 of the $\alpha$ subunit, and the hydrogen bond therebetween has been reported to increase the activation energy for the movement of the $C_2$-OH group (Bilic, L., et al., The Journal of Physical Chemistry B, 123(29), 6178-6187. (2019)). Consequently, the inactivation of pro-S is considered to occur before hydrogen recombination (FIG. 3).

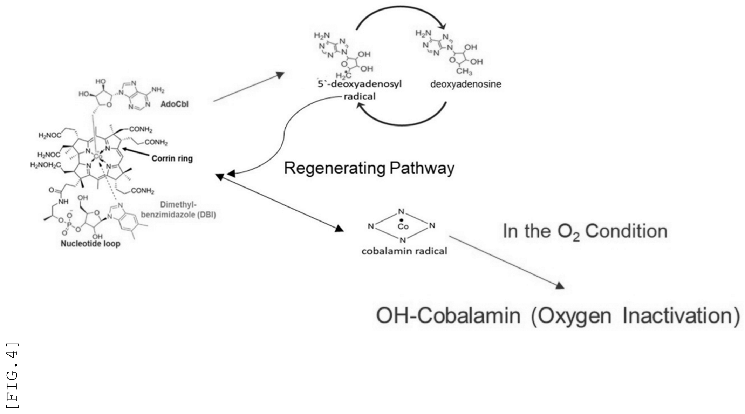

On the other hand, glycerol dehydratase exhibits enzyme activity through the radical reaction of coenzyme B12. When oxygen binds to the cobalt atom of coenzyme B12 during the enzyme radical reaction, the recombination of cobalt to the adenosyl radical is blocked after the reaction is completed, resulting in inactivation. When cobalamin is inactivated, the adenosyl radical cannot be regenerated and remains tightly bound to the active site, making replacement with a new coenzyme impossible and resulting in loss of enzymatic activity (FIG. 4). During introduction of glycerol dehydratase in industrial applications, studies on enzyme variants resistant to inactivation have been reported (Toraya, T., Cellular and Molecular Life Sciences CMLS, 57, 106-127. (2000)).

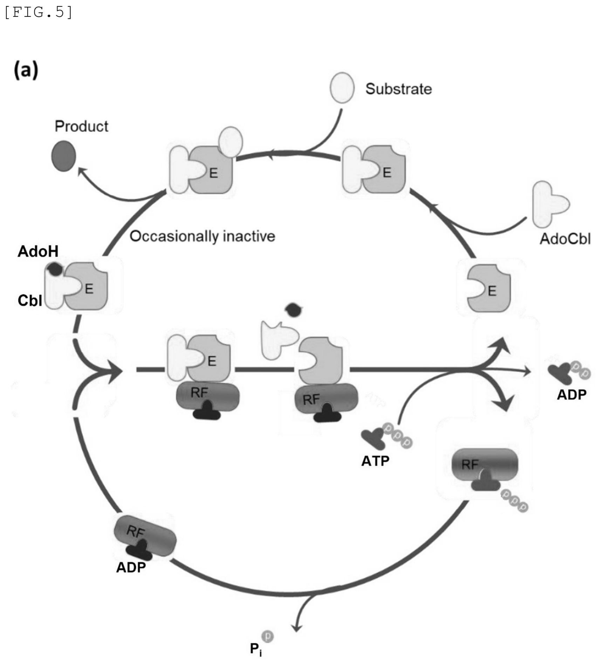

Glycerol dehydratase inactivated by glycerol or oxygen may be reactivated by glycerol dehydratase reactivase. Glycerol dehydratase reactivase releases the inactive coenzyme B12 present in the inactivated enzyme and allows new coenzyme B12 to bind to the active site, restoring enzyme activity. The structural similarity between the β subunit of glycerol dehydratase and the β subunit of glycerol dehydratase reactivase led to the hypothesis that the β subunit of the reactivase replaces the β subunit of glycerol dehydratase, releasing the inactive coenzyme B12 between the α and β subunits (FIG. 5) (Liao, D. I., et al., Structure, 11(1), 109-119. (2003); Shibata, N., et al., Structure, 13(12), 1745-1754. (2005); Tobimatsu, T., et al., Archives of microbiology, 174, 81-88. (2000)).

Studies have been conducted from various perspectives to address this enzyme inactivation problem. To improve the enzyme, there have been attempts to introduce amino acid mutations into the active site of the enzyme whose structure has been elucidated (Yamanishi, M., et al., The FEBS journal, 279(5), 793-804. (2012)). This study showed the presence of Ser301 and Gln335, which play an important role in distinguishing the pro-S and pro-R forms, and when these were each replaced with alanine, the inactivation rate of glycerol was lower than that of the wild type. However, in this case, the enzyme activity was also reported to decrease. Alternatively, there was an attempt to mutate the amino acids Thr172 and Ser224, which are involved in hydrogen bond formation after radical formation of the adenosyl group (Shibata, N., et al., Angewandte Chemie, 130(26), 7956-7961. (2018)). In this case, the inactivation rate of glycerol was also lower than that of the wild type, but the enzyme activity was decreased.

On the other hand, studies focusing on the linkage of glycerol dehydratase α and β subunits have been reported. Glycerol dehydratase (KpGDHt) derived from *K. pneumoniae* has a heterotrimer dimer ((αβγ)2) shape and consists of two α subunits linked to each other. The β and γ subunits each interact with the α subunit. Each αβγ-heterotrimer contains a coenzyme B12 molecule between the α and β subunits (Liao, D. I., et al., Journal of inorganic biochemistry, 93(1-2), 84-91. (2003); Yamanishi, M., et al., European journal of biochemistry, 269(18), 4484-4494. (2002)).

Conventional glycerol dehydrogenase purification processes have been plagued by problems caused by the dissociation of the β subunit. To overcome this, studies have been conducted to link the α subunit to the β subunit (Wang, X. D., et al., Biotechnol Lett, 31(5), 711-717. (2009)). In this study, the α and β subunits of KpGDHt were linked using a $(Gly_4Ser)_4$ linker containing 20 amino acids, and the resulting mutant exhibited catalytic activity ($k_{cat}/K_m$) similar to that of the wild type. In another study, a $G(PT)_4T(PT)_7G$ linker was adopted from endoglucanase A from *Cellulomanas fimi,* and the mutant unexpectedly exhibited a 20°C increase in the optimal activity temperature for 1,2-propanediol (Maddock, D. J., et al., Biotechnol J, 12(12). (2017)).

In another study, in an attempt to engineer KpGDHt to improve the resistance to inactivation, a mutant having a linkage between α and β subunits was constructed by changing the stop codon (TAA) at the end of the α subunit to glutamine (CAA) (FIG. 6) (Gibson et al. U.S. Patent No. 8,569,469 (2013)). This mutant exhibited a slower inactivation rate than the wild-type enzyme in assays using cell lysates.

Under this background, as a result of extensive efforts to develop a glycerol dehydratase with improved glycerol activity and/or oxygen resistance, the present inventors have developed a glycerol dehydratase mutant that improves both the glycerol activity and oxygen resistance and exhibits high activity even under conditions containing low concentrations of coenzyme B12. Based thereon, the present invention has been completed.

The information disclosed in this Background section is provided only for better understanding of the background of the present invention, and therefore it may not include information that forms the prior art that is already obvious to those skilled in the art.

[Prior art literature]

[Non-patent literature]

(Non-patent literature 1) Droźdźyńska A, et al., BioTechnologia, 92-100 (2011)
(Non-patent literature 2) Valdehuesa, et al., Applied microbiology and biotechnology, 97, 3309-3321. (2013)
(Non-patent literature 3) Mitrea L, et al., Microbial Cell Factories, 16, 1-17. (2017)
(Non-patent literature 4) Toraya T, et al., Archives of Biochemistry and Biophysics, 203(1), 174-180. (1980)
(Non-patent literature 5) Mancia, et al., Structure, 4(3), 339-350. (1996)
(Non-patent literature 6) Shibata N, et al., Structure, 7(8), 997-1008. (1999)
(Non-patent literature 7) Kamachi T, et al., Chemistry-A European Journal, 13(28), 7864-7873. (2007)
(Non-patent literature 8) Poznanskaja A, et al., Archives of Biochemistry and Biophysics, 194(2), 379-386. (1979)
(Non-patent literature 9) Yamanishi M, et al., European journal of biochemistry, 269(18), 4484-4494. (2002)
(Non-patent literature 10) Doitomi K, et al., Bulletin of the Chemical Society of Japan, 87(9), 950-959. (2014)
(Non-patent literature 11) Bilić L, et al., The Journal of Physical Chemistry B, 123(29), 6178-6187. (2019)
(Non-patent literature 12) Toraya T, Cellular and Molecular Life Sciences CMLS, 57, 106-127. (2000)

(Non-patent literature 13) Liao D I, et al., Structure, 11(1), 109-119. (2003)
(Non-patent literature 14) Shibata N, et al., Structure, 13(12), 1745-1754. (2005)
(Non-patent literature 15) Tobimatsu T, et al., Archives of microbiology, 174, 81-88. (2000)
(Non-patent literature 16) Yamanishi M, et al., The FEBS journal, 279(5), 793-804. (2012)
(Non-patent literature 17) Shibata N, et al., Angewandte Chemie, 130(26), 7956-7961. (2018)
(Non-patent literature 19) Liao D I, et al., Journal of inorganic biochemistry, 93(1-2), 84-91. (2003)
(Non-patent literature 20) Wang X D, et al., Biotechnol Lett, 31(5), 711-717. (2009)
(Non-patent literature 21) Maddock D J, et al., Biotechnol J, 12(12). (2017)

[Patent literature]

(Patent literature 1) Gibson, et al., U.S. Patent No 8,569,469. (2013)

## SUMMARY OF THE INVENTION

It is one object of the present invention to provide a fused glycerol dehydratase variant that exhibits enhanced oxygen resistance and glycerol activity, and thus exhibits excellent activity even under low concentrations of coenzyme B12.

It is another object of the present invention to provide a nucleic acid encoding the fused glycerol dehydratase variant, a recombinant vector containing the nucleic acid, a recombinant microorganism transformed with the nucleic acid or recombinant vector, and a method of producing 3-hydroxypropionic acid or 1,3-propanediol using the recombinant microorganism.

In accordance with one aspect of the present invention, the above and other objects can be accomplished by the provision of a recombinant glycerol dehydratase (GDHt) variant including a fusion of an alpha subunit and a beta subunit of glycerol dehydratase, wherein the variant includes a substitution of at least one amino acid residue selected from the group consisting of: G19, L29, A177, A207, Y271, and V455 of the alpha subunit represented by SEQ ID NO: 1; and L113, S149, and M158 of the beta subunit represented by SEQ ID NO: 2.

In accordance with another aspect of the present invention, provided is a nucleic acid encoding the fused glycerol dehydratase variant and a recombinant vector containing the nucleic acid.

In accordance with another aspect of the present invention, provided is a recombinant microorganism transformed with the nucleic acid or recombinant vector.

In accordance with another aspect of the present invention, provided is a method of producing 3-hydroxypropionic acid, the method including: culturing the recombinant microorganism in a medium containing glycerol to produce 3-hydroxypropionic acid; and obtaining the produced 3-hydroxypropionic acid.

In accordance with another aspect of the present invention, provided is a method of producing 1,3-propanediol, the method including: culturing the recombinant microorganism in a medium containing glycerol to produce 1,3-propanediol; and obtaining the produced 1,3-propanediol.

## BRIEF DESCRIPTION OF DRAWINGS

FIG. 1 illustrates a pathway for producing 3-HPA from glycerol by glycerol dehydratase and for producing 3-HP and 1,3-PDO from the 3-HPA.

FIG. 2 is a schematic diagram illustrating a reaction mechanism of glycerol dehydratase.

FIG. 3 illustrates two pathways and the corresponding stereostructures of the reaction depending on the position of hydrogen abstracted in the reaction by diol dehydratase and a similar enzyme to glycerol dehydratase.

FIG. 4 is a schematic diagram illustrating the inactivation of glycerol dehydratase in the presence of oxygen and reactivation of glycerol dehydratase.

FIG. 5 illustrates the mechanism of the reactivation reaction of glycerol dehydratase.

FIG. 6 illustrates the structure of a mutant (fGDHt) in which the α subunit and the β subunit are linked to each other by changing the stop codon (TAA) at the end of the α subunit of glycerol dehydratase (KpGDHt) from *Klebsiella pneumoniae* to the codon for glutamine (CAA).

FIG. 7 shows the oxygen resistance and glycerol activity of the purified mutant enzyme, in which the α and β subunits are linked.

FIG. 8 shows the result of comparative protein structure network analysis (PSNA) between the structures of WT kpGDHt having α and β subunits separated from each other, and fGDHt having α and β subunits linked to each other, in an energy-stabilized state confirmed through molecular dynamics simulation.

FIG. 9 is a schematic diagram illustrating a process of selecting candidate mutants in the present invention. The candidates were selected by analyzing the energy stability of all mutants excluding residues common to other species using Rosetta Design (http:/rosettadesign.med.unc.edu/).

FIG. 10 is a graph showing the activity of the wild-type and mutant enzymes measured using the 3-methyl-2-benzothiazolinone hydrazone (MBTH) method.

FIG. 11 is a graph showing oxygen resistance determined by measuring activity using MBTH after exposure of wild-type and mutant enzymes to oxygen for 5 minutes.

FIG. 12 is a graph showing the relative activity of wild-type GDHt, wild-type fGDHt, and variants of the present invention as a function of oxygen exposure time.

FIG. 13 is a graph showing the production of 3-HP recombinant microorganisms containing nucleic acids encoding wild-type GDHt, wild-type fGDHt, and variants of the present invention cultured under various concentrations of coenzyme B12.

## DETAILED DESCRIPTION AND PREFERRED EMBODIMENTS OF THE INVENTION

Unless defined otherwise, all technical and scientific terms used herein have the same meanings as appreciated by those skilled in the field to which the present invention pertains. In general, the nomenclature used herein is well-known in the art and is ordinarily used.

Through analysis of the stereostructure of previously reported fused glycerol dehydratase having a structure in which alpha- and beta-subunits are linked to each other, the present inventors identified the energetic stability of amino acids at the alpha- or beta-subunit interface excluding amino acids that directly interact with coenzymes. In further research, the present inventors identified glycerol dehydratase variants with enhanced enzyme activity, superior oxygen resistance, and enhanced 3-hydroxypropionic acid production under low coenzyme B12 conditions.

Based thereon, the present invention provides a glycerol dehydratase variant including at least one amino acid modification.

The present invention provides a variant that is more resistant to enzyme inactivation by substrate glycerol or oxygen than fused glycerol dehydratase. The mutation is located at the interface between the alpha and beta subunits and includes modifications of amino acid residues that play a key role in the activity and/or reactivation of glycerol dehydratase.

The present invention provides a series of processes for providing the variant including predicting a protein structure in silico methods, visualizing protein-protein binding, predicting protein-protein binding, and visualizing the three-dimensional binding structure.

In one aspect, the present invention is directed to a recombinant glycerol dehydratase (GDHt) variant including a fusion of an alpha subunit and a beta subunit of glycerol dehydratase,

wherein the variant includes a substitution of at least one amino acid residue selected from the group consisting of:

G19, L29, A177, A207, Y271, and V455 of the alpha subunit represented by SEQ ID NO: 1; and

L113, S149, and M158 of the beta subunit represented by SEQ ID NO: 2.

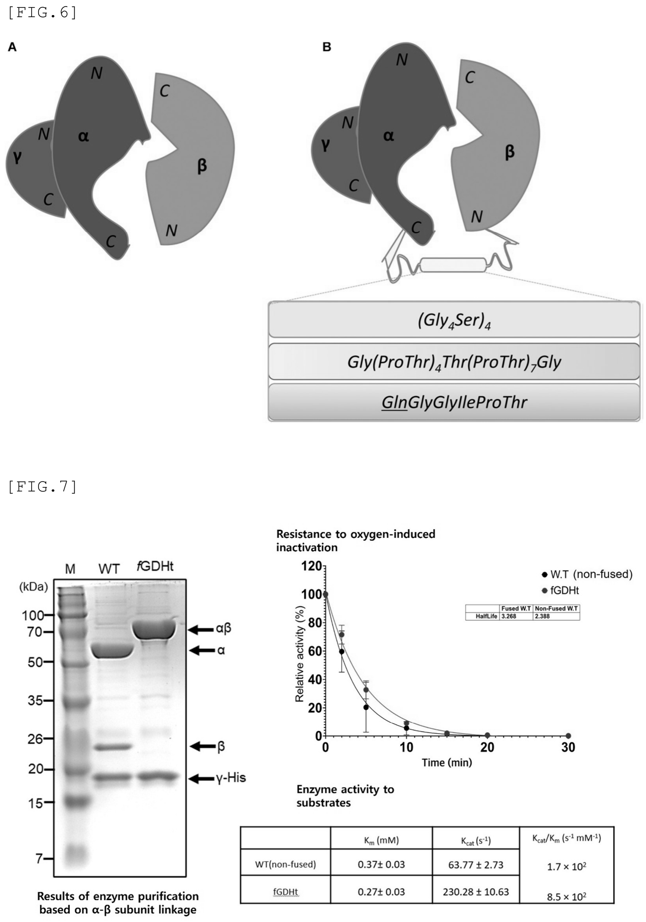

Glycerol dehydratase belongs to the class of hydrolases that cleave carbon-oxygen bonds and may be used interchangeably with the term "glycerol hydrolase". Glycerol dehydratase has a quaternary structure containing two each of three types of subunits: alpha-subunit, beta-subunit, and gamma-subunit (see FIG. 6). Glycerol dehydratase catalyzes the following reaction using cobalamin (B12) as a coenzyme:

$$\text{glycerol} \rightleftharpoons \text{3-hydroxypropanal} + H_2O$$

"Glycerol dehydratase", "GDHt", "glycerol dehydratase", "wild-type GDHt" or "wt GDHt" refers to the wild-type GDHt of *Klebsiella pneumoniae,* unless otherwise specified. The term encompasses untreated GDHt and any form of GDHt obtained by treatment of cells.

In one embodiment, the alpha subunit of the wild-type glycerol dehydratase includes an amino acid sequence represented by SEQ ID NO: 1. The beta subunit of the wild-type glycerol dehydratase includes the amino acid sequence represented by SEQ ID NO: 2. The gamma subunit of the wild-type glycerol dehydratase includes the amino acid sequence represented by SEQ ID NO: 3.

In one embodiment, the present invention provides a variant including an additional modification in a recombinant glycerol dehydratase including an alpha-subunit and a beta-subunit fused to each other.

In one embodiment, the recombinant glycerol dehydratase includes a gamma-subunit having the same sequence as the gamma-subunit of wild-type glycerol dehydratase, and an alpha, beta-fused subunit including an alpha-subunit and a beta-subunit linked to each other and having the same sequence as the wild-type glycerol dehydratase.

Specifically, the variant may include a substitution of at least one residue selected from the group consisting of G19, L29, A177, A207, Y271, and V455 represented by the sequence of SEQ ID NO: 1.

The variant may include at least one substitution selected from the group consisting of A207W($\alpha$), A177M($\alpha$), G19A($\alpha$), L29F($\alpha$), Y271W($\alpha$), and V455W($\alpha$).

Specifically, the variant may include a substitution of at least one residue selected from the group consisting of L113, S149, and M158 represented by the sequence of SEQ ID NO: 2.

The variant may include at least one substitution selected from the group consisting of L113Y($\beta$), L113F($\beta$), S149I($\beta$), L113W($\beta$), S149M($\beta$), M158W($\beta$), and M158Y($\beta$).

The variant may include any one substitution selected from the group consisting of:

A177M($\alpha$), L113W($\beta$), and M158W($\beta$);

A177M($\alpha$) and L113W($\beta$); and

A177M($\alpha$) and M158W($\beta$).

In a specific embodiment, the variant may include an amino acid sequence selected from the group consisting of SEQ ID NOs: 5 to 12.

The term "glycerol dehydratase variant", "vt GDHt", or "mutant GDHt" encompasses any mutant of a GDHt molecule having a configuration in which GDHt is linked to another molecule such as by fusion or chemical conjugation. The mutant has at least one amino acid mutation that may affect the interaction of glycerol dehydratase with coenzyme B12 and/or a glycerol substrate. The mutation may include substitutions, deletions, truncations, or modifications of wild-type amino acid residues normally located at the positions thereof. Mutants obtained by amino acid substitutions are preferred. Unless otherwise specified, the glycerol dehydratase variant may be referred to herein as a GDHt mutant peptide sequence, a GDHt mutant polypeptide, a GDHt mutant protein, or a GDHt variant.

Amino acid positions of the GDHt protein and sequence fragment thereof, the alpha-subunit, the beta-subunit, or the alpha, beta-fusion subunit of GDHt, the alpha-subunit, the beta-subunit, or the alpha, beta-fusion subunit of recombinant GDHt according to the present invention, the amino acid sequences of the alpha-subunit, are referred to with reference to the amino acid sequences of the alpha-subunit, the beta-subunit, and the alpha, beta-fusion subunit of wild-type GDHt. For example, the corresponding amino acid position corresponding to the GDHt alpha-subunit may be identified by the alignment of the amino acid sequence with SEQ ID NO: 1, the corresponding amino acid position corresponding to the GDHt beta-subunit may be identified by the alignment of the amino acid sequence with SEQ ID NO: 2, and the corresponding amino acid position corresponding to the GDHt alpha, beta-fusion subunit may be identified by the alignment of amino acid sequence with SEQ ID NO: 4. Accordingly, unless otherwise stated herein, the glycerol dehydratase and the amino acid positions of the alpha-subunit, beta-subunit, and alpha, beta-fusion subunit thereof correspond to amino acid positions numbered according to SEQ ID NO: 1, SEQ ID NO: 2, and SEQ ID NO: 4, respectively. In this case, a specific residue may be referred to as [amino acid residue/position] and may be referred to as [amino acid residue/position/(subunit)] considering that the GDHt of the present invention has a quaternary structure including three types of subunits. The amino acid residue may be expressed by a three-letter abbreviation and a one-letter abbreviation. For example, "Ala177($\alpha$)" or "A177($\alpha$)" refers to an alanine residue A at position 177 of the alpha subunit of SEQ ID NO: 1, or an amino acid residue at the corresponding position in the polypeptide sequence of another alpha subunit of GDHt by alignment. To perform sequence alignment to determine amino acid positions, the basic local alignment search tool, which is available at https://blast.ncbi.nlm.nih.gov/Blast.cgi, may be used with default parameters.

The names of various forms of GDHt variants may be used herein to refer to the same mutation. When referring to GDHt variant proteins herein, a single amino acid substitution is expressed by as [original amino acid residue/position/-

substituted amino acid residue]. Further, since the GDHt of the present invention has a quaternary structure including three subunits, the subunits are referred to as [original amino acid residue/position/substituted amino acid residue/(subunit)] to specify the subunit. For example, a mutation from alanine to methionine at position 177 may be referred to as A42, Ala42, Ala42, A177M, or Ala42Met. For example, a mutation from alanine to methionine at position 177 of the alpha subunit represented by SEQ ID NO: 1 may be referred to as A42($\alpha$), Ala42($\alpha$), Ala 42($\alpha$), A177M($\alpha$), or Ala42Met($\alpha$). Single amino acid substitutions may be combined together using a comma "," to represent combination variants at multiple given positions.

As used herein, the term "variation" refers to a modification of a protein including the replacement of an amino acid with an amino acid having similar biochemical properties without causing loss of the structural, biological, or biochemical functions of proteins. An "amino acid substitution" refers to a substitution that maintains the structure of the amino acid, but replaces the amino acid with another amino acid having a side chain with different chemical properties. Naturally occurring amino acids may be categorized into amino acids with side chains having similar properties, which is well known. These categories include amino acids with basic side chains (e.g., lysine, arginine, and histidine), amino acids with acidic side chains (e.g., aspartic acid and glutamic acid), amino acids with uncharged polar side chains (e.g., glycine, asparagine, glutamine, serine, threonine, tyrosine, and cysteine), amino acids with nonpolar side chains (e.g., alanine, valine, leucine, isoleucine, proline, phenylalanine, methionine, and tryptophan), amino acids with beta-branched side chains (e.g., threonine, valine, and isoleucine), and amino acids with aromatic side chains (e.g., tyrosine, phenylalanine, tryptophan, and histidine). The present invention provides GDHt variants constructed through an amino acid substitution strategy based on *Klebsiella pneumoniae* GDHt, which demonstrates that the GDHt variants can still retain activity.

The GDHt variants according to the present invention may include not only the sequences of the GDHt variants described herein, but also biological equivalents thereof, as long as they maintain the desired function. For example, additional changes may be made to the amino acid sequence of the protein to further improve the physical properties or other biological characteristics of GDHt. Such modifications include, for example, deletions, insertions, and/or substitutions of amino acid sequence residues in the protein. Specifically, as described above, these modifications are based on the relative similarity of amino acid residue substitutions, such as hydrophobicity, hydrophilicity, charge, and size. Analysis of the size, shape, and type of amino acid residue substitutions reveals that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine may be considered biologically functional equivalents.

Taking into consideration variations having biologically equivalent activity, the GDHt variant according to the present invention is interpreted to include a sequence having substantial identity with the sequence represented by SEQ ID NO. The term "substantial identity" means that a sequence has a homology of at least 90%, most preferably a homology of at least 95%, at least 96%, at least 97%, at least 98% or at least 99%, when aligning the sequence of the present invention with any other sequence so as to correspond to each other as closely as possible and analyzing the aligned sequence using algorithms commonly used in the art. Alignment methods for sequence comparison are well-known in the art. The NCBI basic local alignment search tool (BLAST) is accessible through NCBI or the like, and may be used in conjunction with sequence analysis programs such as BLASTP, BLASTM, BLASTX, TBLASTN and TBLASTX over the Internet. BLAST is available at www.ncbi.nlm.nih.gov/BLAST/. A method of comparing sequence homology using this program may be found at www.ncbi.nlm.nih.gov/BLAST/blast help.html.

Based on this, each subunit of the GDHt variant of the present invention may have a homology of 93%, 94%, 95%, 96%, 97%, 98%, 99%, or more of the sequences of SEQ ID NOS. 1 to 4 disclosed herein. Homology may be determined through sequence comparison and/or alignment by methods known in the art. For example, the percentage sequence homology of the nucleic acid or protein according to the present invention may be determined using a sequence comparison algorithm (*i.e.,* BLAST or BLAST 2.0), manual alignment, or visual inspection.

In particular, the present inventors explored a variant that has maximized enzyme properties by applying additional mutations to a previously reported recombinant glycerol dehydratase including linked alpha- and beta-subunits. It is expected that the degradation of the beta-subunit will be reduced using a recombinant glycerol dehydratase including fused alpha- and beta-subunits, thereby improving resistance to GDHt inactivation. Furthermore, this property can be maximized through additional amino acid substitutions at the interface between the alpha- and beta-subunits.

In one embodiment, the recombinant glycerol dehydratase may be formed into a fusion protein variant by adding amino acid mutations to wild-type GDHt to design a GDHt mutant.

In certain embodiments, the glycerol dehydratase variant including an alpha-subunit and a beta-subunit fused to each other may be produced with reference to the literature, such as Wang X D, et al., Biotechnol Lett, 31(5), 711-717. (2009); Maddock D J, et al., Biotechnol J, 12(12). (2017); Gibson, et al., U.S. Patent No. 8,569,469. (2013). In one embodiment, a recombinant GDHt in which the alpha-subunit and beta-subunit are fused to each other may be produced by changing the last stop codon (TAA) of the alpha-subunit to glutamine (CAA). Further, those skilled in the art may produce recombinant glycerol dehydratase variants including an alpha-subunit and a beta-subunit fused to each other using a method known in the art for linking two different proteins.

As used herein, the term "fusion" refers to the integration of two molecules having different or identical functions or structures, and, for example, includes fusion through any physical, chemical or biological method. The fusion may preferably be carried out using a linker peptide and the linker peptide may mediate the fusion with the bioactive molecule at various positions of the alpha-subunit and beta-subunit of glycerol dehydratase according to the present invention.

Specifically, the alpha subunit and the beta subunit may be linked via a linker.

The alpha subunit of the glycerol dehydratase variant may be linked to the beta subunit via a linker. The linker may be a peptide linker and may have a length of about 10-25 amino acids. For example, the linker may include, but is not limited to, a hydrophilic amino acid such as glycine and/or serine.

Specifically, the linker may include any one selected from the group consisting of, but is not limited to:

$(G_4S)_a$, $G(PT)_bT(PT)_cG$, and QGGIPV,
(wherein a is an integer from 1 to 8, and b and c are integers from 2 to 10).

Specifically, the linker is positioned between the C-terminus of the alpha subunit and the N-terminus of the beta subunit, but is not limited thereto. Any linker that is expected to resolve the enzyme inactivation problem through the linkage of the alpha subunit and the beta subunit falls within the scope of the present invention.

A mutant of wild-type GDHt was designed to enhance oxygen resistance and structural stability of GDHt through amino acid mutation. When the alpha and beta subunits are linked to form a recombinant glycerol dehydratase mutant, the expression amount of the fusion protein increases and the biological properties are improved to address the decrease in enzyme activity due to beta subunit degradation.

In another aspect, the present invention is directed to a nucleic acid encoding the fused glycerol dehydratase variant. The fused glycerol dehydratase variant may be recombinantly produced by isolating the nucleic acid encoding the fused glycerol dehydratase variant of the present invention. One skilled in the art will be able to recombinantly produce the alpha, beta, and gamma subunits of glycerol dehydratase separately or from a single nucleic acid, based on the disclosure provided herein.

The term "nucleic acid" is intended to encompass both DNA (gDNA and cDNA) and RNA molecules, and a nucleotide, which is a basic constituent unit of a nucleic acid, includes naturally derived nucleotides as well as analogues, wherein sugar or base moieties are modified. The sequence of the nucleic acid encoding heavy- and light-chain variable regions of the present invention may vary. Such variation includes addition, deletion, or non-conservative or conservative substitution of nucleotides.

In a specific embodiment, the nucleic acid encoding the variant may include a base sequence selected from the group consisting of SEQ ID NOs: 17 to 24.

The DNA encoding the glycerol dehydratase variant (subunits and fusion subunits) may be easily isolated or synthesized using conventional molecular biological techniques (for example, by using an oligonucleotide probe that may specifically bind to CH3A (EW) and CH3B (RVT) DNA), and the nucleic acid is isolated, inserted into a replicable vector, further cloned (amplified with DNA) or further expressed. Based on this, in another aspect, the present invention is directed to a recombinant expression vector containing the nucleic acid.

As used herein, the term "expression vector" or "vector" refers to a means for expressing target genes in host cells, and includes plasmid vectors, cosmid vectors, and viral vectors such as bacteriophage vectors, adenovirus vectors, retroviral vectors and adeno-associated viral vectors. Vector components generally include, but are not limited to, one or more of the following components: signal sequences, replication origins, one or more antibiotic resistance marker genes, enhancer elements, promoters, and transcription termination sequences. The nucleic acid encoding the glycerol dehydratase variant is operably linked to a promoter and a transcription termination sequence.

The term "operably linked" means functional linkage between a nucleic acid expression regulation sequence (e.g., an array of the binding site of the promoter, signal sequence or transcription regulator) and another nucleic acid sequence, and enables the regulation sequence to regulate the transcription and/or translation of the other nucleic acid sequence.

When a prokaryotic cell is used as a host, it generally includes a potent promoter capable of inducing transcription (such as a tac promoter, lac promoter, lacUV5 promoter, lpp promoter, pL$\lambda$ promoter, pR$\lambda$ promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it includes a promoter derived from the genome of mammalian cells (e.g., a metallothionein promoter, a $\beta$-actin promoter, a human hemoglobin promoter or a human muscle creatine promoter), or a promoter derived from a mammalian virus (e.g., an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter), and generally has a polyadenylation sequence as a transcription termination sequence.

In another aspect, the present invention is directed to a recombinant microorganism transformed with the recombinant expression vector. The recombinant microorganism used to produce the variant of the present invention may be a prokaryote, yeast or higher eukaryotic cell, but is not limited thereto.

Prokaryotic host cells such as *Escherichia coli,* strains of the genus *Bacillus,* such as *Bacillus subtilis* and *Bacillus thuringiensis, Streptomyces spp., Pseudomonas spp.* (for example, *Pseudomonas putida*), *Proteus mirabilis* and *Staphylococcus spp.* (for example, *Staphylococcus carnosus*) may be used.

Interest in animal cells is the greatest, and examples of useful host cell lines include, but are not limited to, COS-7, BHK, CHO, CHOK1, DXB-11, DG-44, CHO/- DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

There is industrial demand for a method of producing 3-hydroxypropionic acid from glycerol and the reaction is illustrated in FIG. 1. To produce 3-hydroxypropionic acid, the recombinant microorganism should express the glycerol dehydratase enzyme aldehyde dehydrogenase.

In one embodiment, a recombinant microorganism may be used in which the glycerol dehydratase variant of the present invention is introduced into a host cell endogenously expressing aldehyde dehydrogenase. Examples of the host cell endogenously expressing aldehyde dehydrogenase include *Escherichia coli* (*E. coli*), *Halobacterium volcanii, Acinetobacter baylyi, Saccharolobus solfataricus,* and *Azospirillum brasilense.*

In another embodiment, even when using a host cell that does not endogenously express aldehyde dehydrogenase, a nucleic acid encoding aldehyde dehydrogenase may be introduced into the host cells to cause expression of aldehyde dehydrogenase. Examples of the nucleic acid encoding aldehyde dehydrogenase include aldh, ALDH1, araE, LhpG, proA, puuC, ALDH3A2, ALDH3A1, ALDH1A1, and the like.

In another embodiment, even when using a host cell that endogenously expresses aldehyde dehydrogenase, a nucleic acid encoding aldehyde dehydrogenase may be introduced into the host cell to increase aldehyde dehydrogenase expression.

In one embodiment of the present invention, a recombinant *E. coli* strain containing a vector expressing the glycerol dehydratase variant of the present invention and a vector expressing alpha-ketoglutaric semialdehyde dehydrogenase (KGSADH) from *Azospirillum brasilense* as the aldehyde dehydrogenase was used as the parent strain. However, any strain expressing aldehyde dehydrogenase may be used without limitation and such strain includes strains expressing exogenously introduced aldehyde dehydrogenase.

As used herein, the term "aldehyde dehydrogenase" refers to an enzyme that catalyzes the reaction that oxidizes 3-hydroxypropionaldehyde to produce 3-hydroxypropionic acid.

The aldehyde dehydrogenase may be 3-hydroxypropionaldehyde dehydrogenase, succinic semialdehyde dehydrogenase, or gamma-glutamyl-gamma-aminobutyraldehyde dehydrogenase, preferably alpha-ketoglutaric semialdehyde dehydrogenase (KGSADH).

Based thereon, the recombinant microorganism may further express an aldehyde dehydrogenase. The recombinant microorganism may be a microorganism for producing 3-hydroxypropionic acid.

There is industrial demand for a method of producing 1,3-propanediol from glycerol, the reaction of which is illustrated in FIG. 1. To produce 1,3-propanediol through this reaction, the recombinant microorganism should express 1,3-propandiol oxidoreductase (PDOR).

In one embodiment, a recombinant microorganism may be used in which the glycerol dehydratase variant of the present invention is introduced into a host cell endogenously expressing 1,3-propanediol oxidoreductase. Examples of the host cell endogenously expressing 1,3-propanediol oxidoreductase include *Clostridium butyricum, Clostridium pasteurianum, and preferably Escherichia coli, Pseudomonas putida, Klebsiella pneumoniae,* and *Citrobacter freundii.*

In another embodiment, even when using a host cell that does not endogenously express 1,3-propanediol oxidoreductase, a nucleic acid encoding 1,3-propanediol oxidoreductase may be introduced into the host cell to increase the expression thereof. Examples of nucleic acids encoding 1,3-propanediol oxidoreductase include pddh, adhE, dhaT, gldA, kduD and the like.

In another embodiment, even when using a host cell that endogenously expresses 1,3-propanediol oxidoreductase, a nucleic acid encoding aldehyde dehydrogenase may be introduced into the host cells to increase the expression of aldehyde dehydrogenase.

Any strain expressing 1,3-propanediol oxidoreductase may be used to produce 1,3-propanediol without limitation. Such a strain also includes strains expressing exogenously introduced 1,3-propanediol oxidoreductase.

Based thereon, the recombinant microorganism may further express 1,3-propanediol oxidoreductase. The recombinant microorganism may be a microorganism for the production of 1,3-propanediol.

As used herein, the term "1,3-propanediol oxidoreductase" refers to an enzyme that catalyzes the reaction of reducing 3-hydroxypropionaldehyde to produce 1,3-propanediol.

In addition, in order to increase the production yield of 3-hydroxypropionic acid or 1,3-propanediol by maintaining the enzymatic activity of glycerol dehydratase, it is preferable that the recombinant microorganism express a glycerol dehydratase reactivase (GDHt reactivase).

In one embodiment, a recombinant microorganism may be used in which the glycerol dehydratase variant of the present invention is introduced into a host cell endogenously expressing a glycerol dehydratase reactivase. Examples of the host cell endogenously expressing glycerol dehydratase include *Clostridium pasteurianum, preferably Klebsiella pneumoniae, Salmonella Typhimurium, Klebsiella michiganesis, Citrobacter freundii,* and the like.

In another embodiment, even when using a host cell that does not endogenously express glycerol dehydratase reactivase, a nucleic acid encoding glycerol dehydratase reactivase may be introduced into the host cell to induce the expression thereof. Examples of nucleic acids encoding the glycerol dehydratase reactivase include pduG, pduH, gdrA, gdrB, ddrA, ddrB, dhaF and the like.

The glycerol dehydratase variant according to the present invention is a variant having improved resistance to inactivation of the enzyme by oxygen or to inactivation of coenzyme B12, and exhibited a high yield even in a medium containing a low coenzyme B12 level of 0.4 $\mu$M or less when producing 3-hydroxypropionic acid using a microorganism.

In another aspect, the present invention is directed to a method of producing 3-hydroxypropionic acid, the method including: culturing the recombinant microorganism in a medium containing glycerol to produce 3-hydroxypropionic acid; and recovring the produced 3-hydroxypropionic acid.

The method may further include: producing 3-hydroxypropionaldehyde (3-HPA) from glycerol through the activity of glycerol dehydratase; and producing 3-hydroxypropionic acid (3-HP) from 3-hydroxypropionaldehyde (3-HPA) through the activity of aldehyde dehydrogenase.

In the production method, the recombinant microorganism may be cultured in the presence of glycerol at a temperature of about 20 to about 45°C and may be cultured using, alone or in combination, (i) carbohydrates including monosaccharides, such as glucose, sucrose, lactose, fructose, maltose, molasses, starch, or cellulose, (ii) an oil and fat, such as soybean oil, sunflower oil, peanut oil, or coconut fat, (iii) a fatty acid, such as palmitic acid, stearic acid, or linoleic acid, (iv) an alcohol, such as glycerol or methanol, (v) an amino acid, such as L-glutamate or L-valine, (vi) an organic acid, such as acetic acid, and the like, according to known methods. In some cases, a known nitrogen source, phosphorus source, metal salt required for growth, precursors, pH regulator or the like may be included.

The cultured and expressed 3-hydroxypropionic acid may be separated and recovered. The culture solution may be filtered or the expressed 3-hydroxypropionic acid may be separated from the culture solution using a centrifuge or sedimentation device. Pure 3-hydroxypropionic acid may be recovered using additional osmosis or purification methods.

The glycerol dehydratase variant according to the present invention exhibits improved resistance to oxygen-induced enzyme inactivation or coenzyme B12 inactivation, and thus high yields are expected when producing 1,3-propanediol using microorganisms.

A method for producing 1,3-propanediol using microorganisms is disclosed in (R.K. Saxena, et al., Biotechnology Advances, Volume 27, Issue 6, 895-913, (2009)) and those skilled in the art may produce 1,3-propanediol in high yield by referring to the disclosure and the references.

In another aspect, the present invention is directed to a method of producing 1,3-propanediol, the method including: culturing the recombinant microorganism in a medium containing glycerol to produce 1,3-propanediol; and recovering the produced 1,3-propanediol.

The method may further include: producing 3-hydroxypropionaldehyde (3-HPA) from glycerol through the activity of glycerol dehydratase; and producing 1,3-propanediol (1,3-PDO) from 3-hydroxypropionaldehyde (3-HPA) through the activity of 1,3-propanediol oxidoreductase.

The details of the method for producing 3-hydroxypropionic acid may be applied to the culture conditions of the microorganism or the method for isolating and recovering the product.

Hereinafter, the present invention will be described in more detail with reference to the following examples. However, it will be obvious to those skilled in the art that the following examples are provided only for illustration of the present invention and should not be construed as limiting the scope of the present invention.

Example 1: Preparation and characterization of fusion glycerol dehydratase (fGDHt)

Example 1-1. Production of fGDHt

The glycerol dehydratase variant of the present invention with improved oxygen resistance or glycerol activity may be produced based on the naturally occurring glycerol dehydratase (KpGDHt) derived from *Klebsiella pneumoniae.* In the present invention, the variant was developed according to the following steps: changes in enzyme properties (resistance to oxygen inactivation and activity to substrates) caused by the linkage of the glycerol dehydratase $\alpha$ and $\beta$ subunits; selection of variant candidates with improved enzyme stability against oxygen based on sequence and computational analysis; enzyme characterization of the candidate variants.

Based on previously reported data (Gibson et al. U.S. Patent No. 8,569,469 (2013)), a fusion glycerol dehydratase including an $\alpha$ subunit and a $\beta$ subunit linked to each other was prepared and purified (FIG. 6, in which the final stop codon (TAA) of the $\alpha$ subunit was changed to glutamine (CAA)).

The amino acid and base sequences of respective subunits of the natural and fusion GDHts are shown in Tables 1 and 2, respectively.

[Table 1]

| GDHt | Subunit | Amino acid sequence |
|------|---------|---------------------|
| Natural GDHt | α | MKRSKRFAVLAQRPVNQDGLIGEWPEEGLIAMDSPFDP VSSVKVDNGLIVELDGKRRDQFDMIDRFIADYAINVER TEQAMRLEAVEIARMLVDIHVSREEIIAITTAITPAKA VEVMAQMNVVEMMMALQKMRARRTPSNQCHVTNLKDNP VQIAADAAEAGIRGFSEQETTVGIARYAPFNALALLVG SQCGRPGVLTQCSVEEATELELGMRGLTSYAETVSVYG TEAVFTGDDTPWSKAFLASAYASRGLKMRYTSGTGSE ALMGYSESKSMLYLESRCIFITKGAGVQGLQNGAVSCI GMTGAVPSGIRAVLAENLIASMLDLEVASANDQTFSHS DIRRTARTLMQMLPGTDFIFSGYSAVPNYDNMFAGSNF DAEDFDDYNILQRDLMVDGGLRPVTEAETIAIRQKAAR AIQAVFRELGLPPIADEEVEAATYAHGSNEMPPRNVVE DLSAVEEMMKRNITGLDIVGALSRSGFEDIASNILNML RQRVTGDYLQTSAILDRQFEVVSAVNDINDYQGPGTGY RISAERWAEIKNIPGVVQPDTIE (SEQ ID NO.: 1) |
| | β | MQQTTQIQPSFTLKTREGGVASADERADEVVIGVGPAF DKHQHHTLIDMPHGAILKELIAGVEEEGLHARVVRILR TSDVSFMAWDAANLSGSGIGIGIQSKGTTVIHQRDLLP LSNLELFSQAPLLTLETYRQIGKNAARYARKESPSPVP VVNDQMVRPKFMAKAALFHIKETKHVVQDAEPVTLHID LVRE (SEQ ID NO.: 2) |
| | γ | MSEKTMRVQDYPLATRCPEHILTPTGKPLTDITLEKVL SGEVGPQDVRISRQTLEYQAQIAEQMQRHAVARNFRRA AELIAIPDERILAIYNALRPFRSSQAELLAIADELEHT WHATVNAAFVRESAEVYQQRHKLRKGS (SEQ ID NO.: 3) |

(continued)

| GDHt | Subunit | Amino acid sequence |
|---|---|---|
| Fused GDHt | α-β fusion | MKRSKRFAVLAQRPVNQDGLIGEWPEEGLIAMDSPFDP VSSVKVDNGLIVELDGKRRDQFDMIDRFIADYAINVER TEQAMRLEAVEIARMLVDIHVSREEIIAITTAITPAKA VEVMAQMNVVEMMMALQKMRARRTPSNQCHVTNLKDNP VQIAADAAEAGIRGFSEQETTVGIARYAPFNALALLVG SQCGRPGVLTQCSVEEATELELGMRGLTSYAETVSVYG TEAVFTDGDDTPWSKAFLASAYASRGLKMRYTSGTGSE ALMGYSESKSMLYLESRCIFITKGAGVQGLQNGAVSCI GMTGAVPSGIRAVLAENLIASMLDLEVASANDQTFSHS DIRRTARTLMQMLPGTDFIFSGYSAVPNYDNMFAGSNF DAEDFDDYNILQRDLMVDGGLRPVTEAETIAIRQKAAR AIQAVFRELGLPPIADEEVEAATYAHGSNEMPPRNVVE DLSAVEEMMKRNITGLDIVGALSRSGFEDIASNILNML RQRVTGDYLQTSAILDRQFEVVSAVNDINDYQGPGTGY RISAERWAEIKNIPGVVQPDTIE**QGGIPV**QQTTQIQPS FTLKTREGGVASADERADEVVIGVGPAFDKHQHHTLID MPHGAILKELIAGVEEEGLHARVVRILRTSDVSFMAWD AANLSGSGIGIGIQSKGTTVIHQRDLLPLSNLELFSQA PLLTLETYRQIGKNAARYARKESPSPVPVVNDQMVRPK FMAKAALFHIKETKHVVQDAEPVTLHIDLVRE   (SEQ ID NO.: 4) |
| | γ | Same as natural GDHt |

[Table 2]

| GDHt | Subunit | Base sequence |
|---|---|---|
| Natural GDHt | α | ATGAAAAGATCAAAACGATTTGCAGTACTGGCCCAGCGCCC CGTCAATCAGGACGGGCTGATTGGCGAGTGGCCTGAAGAGG GGCTGATCGCCATGGACAGCCCCTTTGACCCGGTCTCTTCA GTAAAAGTGGACAACGGTCTGATCGTCGAGCTGGACGGCAA |

(continued)

| GDHt | Subunit | Base sequence |
|---|---|---|
| | | ACGCCGGGACCAGTTTGACATGATCGACCGATTTATCGCCG ATTACGCGATCAACGTTGAGCGCACAGAGCAGGCAATGCGC CTGGAGGCGGTGGAAATAGCCCGCATGCTGGTGGATATTCA CGTCAGTCGGGAGGAGATCATTGCCATCACTACCGCCATCA CGCCGGCCAAAGCGGTCGAGGTGATGGCGCAGATGAACGTG GTGGAGATGATGATGGCGCTGCAGAAGATGCGTGCCCGCCG GACCCCCTCCAACCAGTGCCACGTCACCAATCTCAAAGATA ATCCGGTGCAGATTGCTGCTGACGCCGCCGAGGCCGGGATC CGCGGCTTCTCAGAACAGGAGACCACGGTCGGTATCGCGCG CTATGCGCCGTTTAACGCCCTGGCGCTGTTGGTCGGTTCGC AGTGCGGCCGCCCCGGCGTTTTGACGCAGTGCTCGGTGGAA GAGGCCACCGAGCTGGAGCTGGGCATGCGTGGCTTAACCAG CTACGCCGAGACGGTGTCGGTCTACGGCACCGAAGCGGTAT TTACCGACGGCGATGATACTCCGTGGTCAAAGGCGTTCCTC GCCTCGGCCTACGCCTCCCGCGGGTTGAAAATGCGCTACAC CTCCGGCACCGGATCCGAAGCGCTGATGGGCTATTCGGAGA GCAAGTCGATGCTCTACCTCGAATCGCGCTGCATCTTCATT ACCAAAGGCGCCGGGGGTTCAGGGGCTGCAAAACGGCGCGGT GAGCTGTATCGGCATGACCGGCGCTGTGCCGTCGGGCATTC GGGCGGTGCTGGCGGAAAACCTGATCGCCTCTATGCTCGAC CTCGAAGTGGCGTCCGCCAACGACCAGACTTTCTCCCACTC GGATATTCGCCGCACCGCGCGCACCCTGATGCAGATGCTGC CGGGCACCGACTTTATTTTCTCCGGCTACAGCGCGGTGCCG AACTACGACAACATGTTCGCCGGCTCGAACTTCGATGCGGA AGATTTTGATGATTACAACATCCTGCAGCGTGACCTGATGG TTGACGGCGGCCTGCGTCCGGTGACCGAGGCGGAAACCATT GCCATTCGCCAGAAAGCGGCGCGGGCGATCCAGGCGGTTTT CCGCGAGCTGGGGCTGCCGCCAATCGCCGACGAGGAGGTGG AGGCCGCCACCTACGCGCACGGTAGCAACGAGATGCCGCCG CGTAACGTGGTGGAGGATCTGAGTGCGGTGGAAGAGATGAT GAAGCGCAACATCACCGGCCTCGATATTGTCGGCGCGCTGA GCCGCAGCGGCTTTGAGGATATCGCCAGCAATATTCTCAAT ATGCTGCGCCAGCGGGTCACCGGCGATTACCTGCAGACCTC GGCCATTCTCGATCGGCAGTTCGAGGTGGTGAGTGCGGTCA ACGACATCAATGACTATCAGGGGCCGGGCACCGGCTATCGC ATCTCTGCCGAACGCTGGGCGGAGATCAAAAATATTCCGGG CGTGGTTCAGCCCGACACCATTGAATAA (SEQ ID NO.: 13) |

(continued)

| GDHt | Subunit | Base sequence |
|---|---|---|
| | β | GTGCAACAGACAACCCAAATTCAGCCCTCTTTTACCCTGAA AACCCGCGAGGGCGGGGTAGCTTCTGCCGATGAACGCGCCG ATGAAGTGGTGATCGGCGTCGGCCCTGCCTTCGATAAACAC CAGCATCACACTCTGATCGATATGCCCCATGGCGCGATCCT CAAAGAGCTGATTGCCGGGGTGGAAGAAGAGGGGCTTCACG CCCGGGTGGTGCGCATTCTGCGCACGTCCGACGTCTCCTTT ATGGCCTGGGATGCGGCCAACCTGAGCGGCTCGGGGATCGG CATCGGTATCCAGTCGAAGGGGACCACGGTCATCCATCAGC GCGATCTGCTGCCGCTCAGCAACCTGGAGCTGTTCTCCCAG GCGCCGCTGCTGACGCTGGAAACCTACCGGCAGATTGGCAA AAACGCCGCGCGCTATGCGCGCAAAGAGTCACCTTCGCCGG TGCCGGTGGTGAACGATCAGATGGTGCGGCCGAAATTTATG GCCAAAGCCGCGCTATTTCATATCAAAGAGACCAAACATGT GGTGCAGGACGCCGAGCCCGTCACCCTGCACGTCGACTTAG TAAGGGAGTGA (SEQ ID NO.: 14) |
| | γ | ATGAGCGAGAAAACCATGCGCGTGCAGGATTATCCGTTAGC CACCCGCTGCCCGGAGCATATCCTGACGCCTACCGGCAAAC CATTGACCGATATTACCCTCGAGAAGGTGCTCTCTGGCGAG GTGGGCCCGCAGGATGTGCGGATCTCCTGCCAGACCCTTGA GTACCAGGCGCAGATTGCCGAGCAGATGCAGCGCCATGCGG TGGCGCGCAATTTCCGCCGCGCGGCGGAGCTTATCGCCATT CCTGACGAGCGCATTCTGGCTATCTATAACGCGCTGCGCCC GTTCCGCTCCTCGCAGGCGGAGCTGCTGGCGATCGCCGACG AGCTGGAGCACACCTGGCATGCGACAGTGAATGCCGCCTTT GTCCGGGAGTCGGCGGAAGTGTATCAGCAGCGGCATAAGCT GCGTAAAGGAAGCTAA (SEQ ID NO.: 15) |

(continued)

| GDHt | Subunit | Base sequence |
|---|---|---|
| Fused GDHt | α-β fusion | ATGAAAAGATCAAAACGATTTGCAGTACTGGCCCAGCGCCC<br>CGTCAATCAGGACGGGCTGATTGGCGAGTGGCCTGAAGAGG<br>GGCTGATCGCCATGGACAGCCCCTTTGACCCGGTCTCTTCA<br>GTAAAAGTGGACAACGGTCTGATCGTCGAGCTGGACGGCAA<br>ACGCCGGGACCAGTTTGACATGATCGACCGATTTATCGCCG<br>ATTACGCGATCAACGTTGAGCGCACAGAGCAGGCAATGCGC<br>CTGGAGGCGGTGGAAATAGCCCGCATGCTGGTGGATATTCA<br>CGTCAGTCGGGAGGAGATCATTGCCATCACTACCGCCATCA<br>CGCCGGCCAAAGCGGTCGAGGTGATGGCGCAGATGAACGTG<br>GTGGAGATGATGATGGCGCTGCAGAAGATGCGTGCCCGCCG<br>GACCCCCTCCAACCAGTGCCACGTCACCAATCTCAAAGATA<br>ATCCGGTGCAGATTGCTGCTGACGCCGCCGAGGCCGGGATC<br>CGCGGCTTCTCAGAACAGGAGACCACGGTCGGTATCGCGCG<br>CTATGCGCCGTTTAACGCCCTGGCGCTGTTGGTCGGTTCGC<br>AGTGCGGCCGCCCCGGCGTTTTGACGCAGTGCTCGGTGGAA<br>GAGGCCACCGAGCTGGAGCTGGGCATGCGTGGCTTAACCAG<br>CTACGCCGAGACGGTGTCGGTCTACGGCACCGAAGCGGTAT<br>TTACCGACGGCGATGATACTCCGTGGTCAAAGGCGTTCCTC<br>GCCTCGGCCTACGCCTCCCGCGGGTTGAAAATGCGCTACAC<br>CTCCGGCACCGGATCCGAAGCGCTGATGGGCTATTCGGAGA<br>GCAAGTCGATGCTCTACCTCGAATCGCGCTGCATCTTCATT<br>ACCAAAGGCGCCGGGGTTCAGGGGCTGCAAAACGGCGCGGT<br>GAGCTGTATCGGCATGACCGGCGCTGTGCCGTCGGGCATTC<br>GGGCGGTGCTGGCGGAAAACCTGATCGCCTCTATGCTCGAC<br>CTCGAAGTGGCGTCCGCCAACGACCAGACTTTCTCCCACTC<br>GGATATTCGCCGCACCGCGCGCACCCTGATGCAGATGCTGC<br>CGGGCACCGACTTTATTTTCTCCGGCTACAGCGCGGTGCCG<br>AACTACGACAACATGTTCGCCGGCTCGAACTTCGATGCGGA<br>AGATTTTGATGATTACAACATCCTGCAGCGTGACCTGATGG<br>TTGACGGCGGCCTGCGTCCGGTGACCGAGGCGGAAACCATT<br>GCCATTCGCCAGAAAGCGGCGCGGGCGATCCAGGCGGTTTT<br>CCGCGAGCTGGGGCTGCCGCCAATCGCCGACGAGGAGGTGG |

(continued)

| GDHt | Subunit | Base sequence |
|---|---|---|
| | | AGGCCGCCACCTACGCGCACGGTAGCAACGAGATGCCGCCG CGTAACGTGGTGGAGGATCTGAGTGCGGTGGAAGAGATGAT GAAGCGCAACATCACCGGCCTCGATATTGTCGGCGCGCTGA GCCGCAGCGGCTTTGAGGATATCGCCAGCAATATTCTCAAT ATGCTGCGCCAGCGGGTCACCGGCGATTACCTGCAGACCTC GGCCATTCTCGATCGGCAGTTCGAGGTGGTGAGTGCGGTCA ACGACATCAATGACTATCAGGGGCCGGGCACCGGCTATCGC ATCTCTGCCGAACGCTGGGCGGAGATCAAAAATATTCCGGG CGTGGTTCAGCCCGACACCATTGAAcAAGGCGGTATTCCTG TGCAACAGACAACCCAAATTCAGCCCTCTTTTACCCTGAAA ACCCGCGAGGGCGGGGTAGCTTCTGCCGATGAACGCGCCGA TGAAGTGGTGATCGGCGTCGGCCCTGCCTTCGATAAACACC AGCATCACACTCTGATCGATATGCCCCATGGCGCGATCCTC AAAGAGCTGATTGCCGGGGTGGAAGAAGAGGGGCTTCACGC CCGGGTGGTGCGCATTCTGCGCACGTCCGACGTCTCCTTTA TGGCCTGGGATGCGGCCAACCTGAGCGGCTCGGGGATCGGC ATCGGTATCCAGTCGAAGGGGACCACGGTCATCCATCAGCG CGATCTGCTGCCGCTCAGCAACCTGGAGCTGTTCTCCCAGG CGCCGCTGCTGACGCTGGAAACCTACCGGCAGATTGGCAAA AACGCCGCGCGCTATGCGCGCAAAGAGTCACCTTCGCCGGT GCCGGTGGTGAACGATCAGATGGTGCGGCCGAAATTTATGG CCAAAGCCGCGCTATTTCATATCAAAGAGACCAAACATGTG GTGCAGGACGCCGAGCCCGTCACCCTGCACGTCGACTTAGT AAGGGAGTGA (SEQ ID NO.: 16) |
| | γ | Same as Natural GDHt |

Example 1-2. Characterization of produced fGDHt

Analysis using purified enzymes revealed that adding a peptide linker between the α and β subunits of kpGDHt increased the resistance of enzyme to oxygen inactivation by approximately 37%, based on half-life. Furthermore, the activity to the glycerol substrate increased approximately five times (FIG. 7).

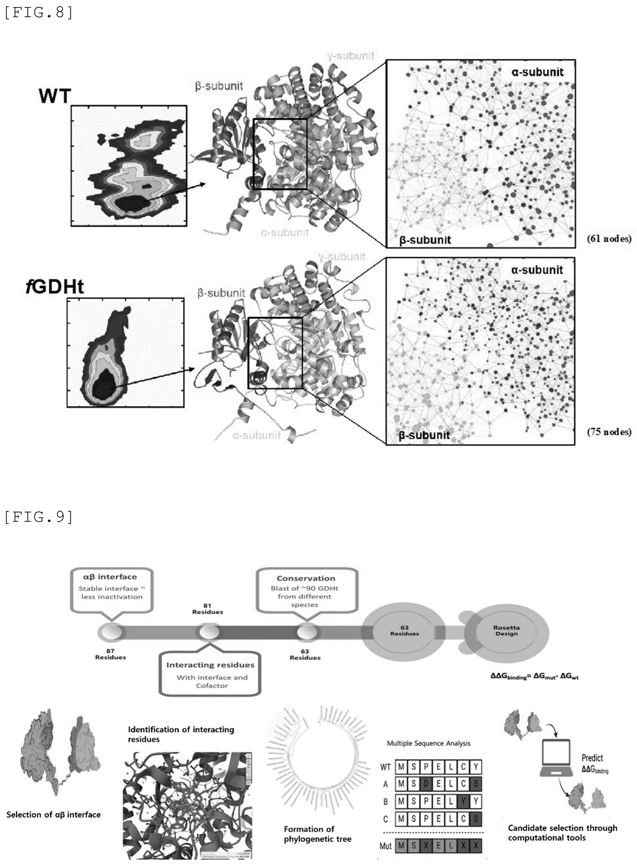

Computer-based structural analysis was performed to determine the mechanism in which the linkage between the α and β subunits affects enzymatic properties. A wild-type *Klebsiella pneumoniae* glycerol dehydratase (WT kpGDHt), whose structure has been elucidated, was produced and a fusion GDHt (fGDHt) was produced using the using the wild-type *Klebsiella pneumoniae* glycerol dehydratase as a template. The energy-stabilized state of a conventional WT kpGDHt including α and β subunits separated from each other, and fGDHt, which has the α and β subunits linked to each other, confirmed by molecular dynamics simulations were compared using protein structure network analysis (PSN). The results revealed that the number of surface-to-surface amino acid interactions between the α and β subunits in fGDHt was increased compared to WT kpGDHt (FIG. 8). This suggests that interactions between the α and β subunits influence enzymic stability and contribute to changes in enzyme properties.

Example 2: Selection of candidate variants based on sequence and computational analysis

To further enhance enzyme stability through amino acid mutations at the interface between the α subunit and the β subunit, sequence analysis of GDHt and computational energy stabilization analysis were performed. First, amino acids were identified at the interface between the α subunit and the β subunit. Specifically, contact surface analysis was performed using Pymol software (see https://pymolwiki.org/index.php/Contact Surface), to calculate the solvent accessible surface area (ASA) when the amino acid is present in each GDHt subunit, and the solvent accessible surface area (ASA) when a quaternary structure is formed. 87 amino acids whose ASA decreased by at least 1Å2 upon formation of the quaternary structure were selected as amino acids at the interface between the α subunit and the β subunit. To alter the interactions between subunits, 81 amino acid residues, excluding 6 residues directly interacting with coenzyme B12 or the substrate, were subjected to multiple sequence alignment (MSA) and then compared with sequence information of other glycerol dehydratases obtained through BLAST. Among the 81 residues, 63 amino acid residues were finally selected by excluding residues that were found as identical amino acids when compared with glycerol dehydratase of other species and thus were determined to be important amino acid residues for enzyme activity (FIG. 9).

19 amino acid variants were selected by excluding the wild type at each amino acid position from the 63 selected amino acid residues, and the energy stability of all variants was analyzed through Rosetta Design (http:/rosettadesign.med.unc.edu/). The top 19 mutant candidates predicted to have low ΔΔG biding energy were finally selected (FIG. 9). Table 3 below shows the analysis results for the 19 variant candidates.

[Table 3]

| Results of energy stability analysis using Rosetta Design | | | |
|---|---|---|---|
| **Mutation** | | **ddG_bind** | **Number of Neighbors** |
| 113 (β) | L>F | -2.1 | 16 |
| 113(β) | L>Y | -2.1 | 16 |
| 113(β) | L>W | -1.9 | 16 |
| 149(β) | S>I | -1.0 | 15 |
| 149(β) | S>M | -0.9 | 15 |
| 19(α) | G>A | -0.7 | 21 |
| 207(α) | A>W | -0.7 | 16 |
| 117(β) | N>Y | -0.6 | 26 |
| 207(α) | A>V | -0.6 | 16 |
| 29(α) | L>F | -0.5 | 26 |
| 158(β) | M>Y | -0.5 | 19 |
| 158(β) | M>W | -0.5 | 19 |
| 177(α) | A>M | -0.5 | 23 |
| 271(α) | Y>W | -0.5 | 14 |
| 455(α) | V>W | -0.5 | 14 |
| 268(α) | L>Y | -0.5 | 18 |
| 158(β) | M>F | -0.5 | 19 |
| 207(α) | A>Y | -0.5 | 16 |
| 207(α) | A>M | -0.5 | 16 |

Example 3: Analysis of enzymatic properties of candidate variants

Example 3-1. Analysis of activity on glycerol

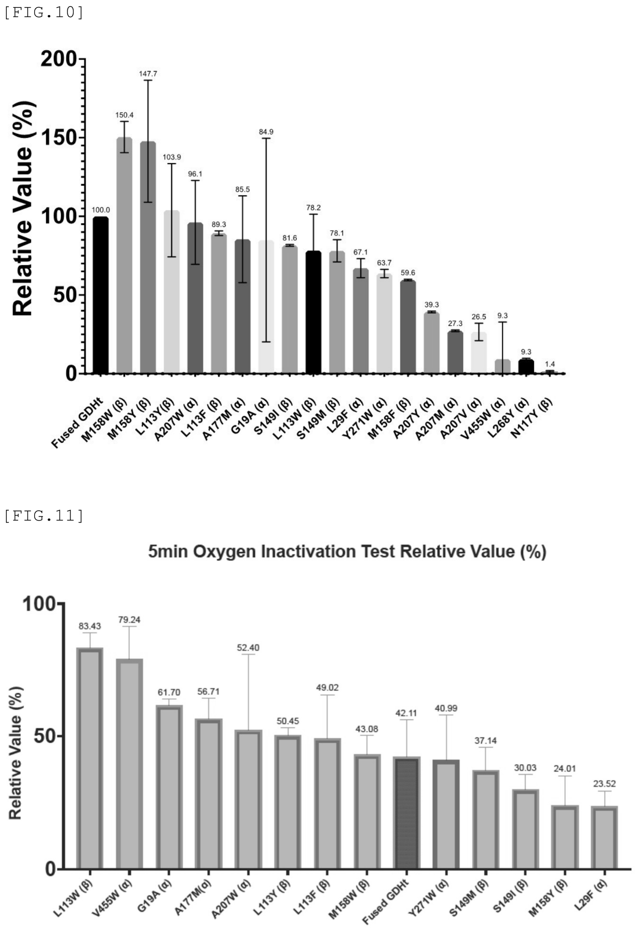

The activities of the wild-type and variant enzymes were measured at 25°C using 3-methyl-2-benzothiazolinone hydrazone (MBTH). To eliminate oxygen inactivation, the enzymes were incubated for 5 minutes in 35 mM phosphate buffer (pH 8.0) stirred with a negative pressure pump. The reaction was initiated by the addition of glycerol. The absorbance measured at 670 nm of the variant was compared with that of the wild-type fGDHt to determine the relative substrate activity (FIG. 10). The MBTH assay was performed as follows:

First, an assay mixture was prepared by mixing glycerol dehydratase, coenzyme B12, 0.05 M potassium chloride (KCl), and 35 mM phosphate buffer (pH 8.0). The reaction was then initiated by adding glycerol, the substrate to the assay mixture. The reaction was performed for 5 minutes and the reaction was terminated by adding 12 mg/mL MBTH and 0.1 M potassium citrate buffer (pH 3.6). Five minutes after the reaction was terminated, $FeCl_3$ was added thereto and the reaction was performed for 15 minutes. The absorbance was measured at 670 nm.

When the enzyme activity to the substrate glycerol of the 19 selected mutant candidates was measured, two variants (M158W(β) and M158Y(β)) exhibited higher activity to glycerol than the wild-type fGDHt. Furthermore, ten variants (L113Y(β), A207W(α), L113F(β), A177M(α), G19A(α), S149I(β), L113W(β), S149M(β), L29F(α), Y271W(α)) exhibited enzyme activity of 60% or more, compared to the wild-type fGDHt. Additional experiments were performed on these 12 variants.

Example 3-2. Oxygen resistance analysis

To observe oxygen-induced inactivation, the enzyme was reacted with coenzyme B12 in 35 mM phosphate buffer (pH 8.0) without removing oxygen for 5 minutes. After the substrate, glycerol, was added, the MBTH assay was performed for 5 minutes (FIG. 11). The assay was performed by calculation as a relative activity with respect to 100% of the activity when the enzyme was not exposed to oxygen. Consequently, 10 variants (L113W(β), V455W(α), G19A(α), A177M(α), A207W(α), L113Y(β), L113F(β), M158W(β), Y271W(α), S149M(β)) exhibiting inactivation resistance higher than or similar to that of the wild-type fGDHt were selected and experiments were conducted to determine the oxygen-dependent half-lives of the selected variants.

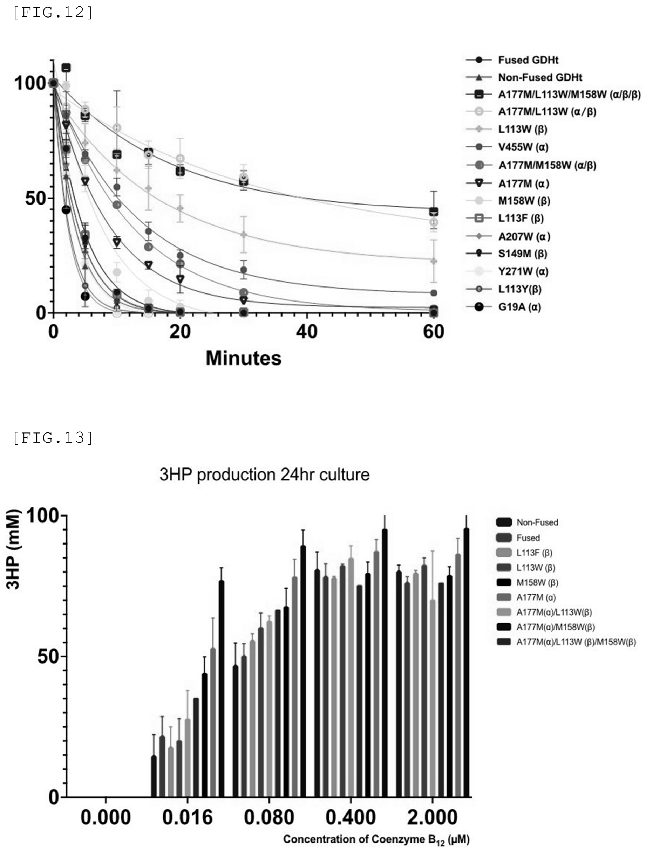

The oxygen-induced inactivation resistance of 10 variants was analyzed and the half-lives thereof were calculated. Inactivation by oxygen was induced by reaction with coenzyme B12 in 35 mM phosphate buffer (pH 8.0) without oxygen removal. Enzyme activity was measured for different oxygen exposure times from 0 to 60 minutes. The relative activity over time was determined based on the activity (100%) of the enzyme without oxygen exposure (exposure for 0 minutes) (FIG. 12). Table 4 below shows the half-lives of each mutant.

[Table 4]

| Half-lives of wild-type GDHt, fGDHt, and variants | |
|---|---|
| **Glycerol dehydratase** | **Half-life (min)** |
| WT | 2.388 |
| fGDHt | 3.268 |
| A177M/L113W/M158W (α/β/β) | 35.06 |
| A177M/L113W (α/β) | 23.32 |
| L113W (β) | 11.78 |
| V455W (α) | 9.068 |
| A177M/M158W(α/β) | 8.643 |
| A177M (α) | 6.021 |
| M158W (β) | 5.144 |
| L113F (β) | 3.284 |
| S149M (β) | 2.78 |
| A207W (α) | 2.78 |
| Y271W (α) | 2.401 |
| L113Y (β) | 1.715 |
| G19A (α) | 1.609 |

As a result, four variants having increased resistance to oxygen inactivation compared to fGDHt were identified: (A177M(α), V455W(α), M158W(β), L113W(β)).

Further, the following variants containing combinations of the amino acid substitutions were constructed and analyzed for enzymatic activity and oxygen resistance: A177M(α)/L113W(β), A177M(α)/M158W(β), L113W(β)/M158W(β), and A177M(α)/L113W(β)/M158W(β). Among the variants, A177M(α)/L113W(β), A177M(α)/M158W(β), and A177M(α)/L113W(β)/M158W(β) exhibited enzymatic activity and oxygen resistance that was 7-times, 2.5-times, and 10.7-times greater than that of fGDHt, respectively.

Example 3-3. Measurement of enzyme reaction rates to substrates of major variants

The variants L113W(β), A177M(α), L113F(β), M158W(β), A177M/L113W(α/β), A177M/M158W(α/β), and A177M/L113W/M158W(α/β/β) with increased oxygen resistance compared to fGDHt found in Example 3-2 were purified and the enzymatic reaction rates thereof with glycerol were measured. To measure the enzymatic reaction rates, an MBTH assay was performed for 5 minutes in 35 mM phosphate buffer (pH 8.0) deoxygenated by a negative pressure pump, 2 μM coenzyme B12, and various concentrations of glycerol.

$K_m$ and $K_{cat}$ were calculated using the following Equation (Michaelis-Menten equation):

[Equation 1]

$$v = \frac{\mathrm{d[P]}}{\mathrm{d}t} = V_{max}\frac{[\mathrm{S}]}{K_M + [\mathrm{S}]} = k_{cat}[\mathrm{E}]_0\frac{[\mathrm{S}]}{K_M + [\mathrm{S}]}$$

wherein $K_m$ is the Michaelis constant and $K_{cat}$ is the enzyme turnover number.

The $K_m$, $K_{cat}$, and $K_{cat}/K_m$ of each variant are shown in Table 5.

[Table 5]

| Enzyme | $K_m$ (mM) | $K_{cat}$ (s⁻¹) | $K_m/K_{cat}$ (s⁻¹ mM⁻¹) |
|---|---|---|---|
| WT | 0.37±0.03 | 63.77±2.73 | $1.7\times10^2$ |
| fGDHt | 0.27±0.03 | 230.28±10.63 | $8.5\times10^2$ |
| fGDHt (L113F(β)) | 0.24±0.06 | 208.62±21.8 | $9.0\times10^2$ |
| fGDHt (L113W(β)) | 0.52±0.17 | 31.82±10.99 | $6.4\times10^2$ |
| fGDHt (A177M (α)) | 0.36±0.07 | 107.48±14.91 | $3.0\times10^2$ |
| FGDHt(M158W(β)) | 0.34±0.05 | 46.38±2. 61 | $1.4\times10^2$ |
| FGDHt(A177M/L113W(α/β)) | 0. 63±0.46 | 31.96±9.42 | $6.3\times10^2$ |
| FGDHt(A177M/M158W(α/β)) | 0.15±0.04 | 155.47±13.23 | $9.2\times10^2$ |
| FGDHt(A177M/L113W/M158W(α/β/β)) | 0.14±0.04 | 28.93±2.17 | $2.1\times10^2$ |

In particular, among the mutants, L113F (β) and A177M/M158W (α/β) had $k_{cat}/K_m$, which are indicators of enzyme reaction rate, that were 6% and 8% higher, respectively, than those of fGDHt. The remaining variants also had sufficiently high $k_{cat}/K_m$.

The amino acid and base sequences of the alpha-beta fusion subunits of each variant used in the experiment are shown in Tables 6 and 7, respectively. The gamma subunits of the mutants are identical to those of native GDHt.

[Table 6]

| Variant | Amino acid sequence of α-β fusion subunit |
|---|---|
| (α)A177M | MKRSKRFAVLAQRPVNQDGLIGEWPEEGLIAMDSPFDPVSSVKVDNGLI VELDGKRRDQFDMIDRFIADYAINVERTEQAMRLEAVEIARMLVDIHVS REEIIAITTAITPAKAVEVMAQMNVVEMMMALQKMRARRTPSNQCHVTN LKDNPVQIAADAAEAGIRGFSEQETTVGI**M**RYAPFNALALLVGSQCGRP GVLTQCSVEEATELELGMRGLTSYAETVSVYGTEAVFTDGDDTPWSKAF LASAYASRGLKMRYTSGTGSEALMGYSESKSMLYLESRCIFITKGAGVQ GLQNGAVSCIGMTGAVPSGIRAVLAENLIASMLDLEVASANDQTFSHSD IRRTARTLMQMLPGTDFIFSGYSAVPNYDNMFAGSNFDAEDFDDYNILQ RDLMVDGGLRPVTEAETIAIRQKAARAIQAVFRELGLPPIADEEVEAAT YAHGSNEMPPRNVVEDLSAVEEMMKRNITGLDIVGALSRSGFEDIASNI LNMLRQRVTGDYLQTSAILDRQFEVVSAVNDINDYQGPGTGYRISAERW AEIKNIPGVVQPDTIE**QGGIPV**QQTTQIQPSFTLKTREGGVASADERAD EVVIGVGPAFDKHQHHTLIDMPHGAILKELIAGVEEEGLHARVVRILRT SDVSFMAWDAANLSGSGIGIGIQSKGTTVIHQRDLLPLSNLELFSQAPL LTLETYRQIGKNAARYARKESPSPVPVVNDQMVRPKFMAKAALFHIKET KHVVQDAEPVTLHIDLVRE (SEQ ID NO.: 5) |
| (α)V455W | MKRSKRFAVLAQRPVNQDGLIGEWPEEGLIAMDSPFDPVSSVKVDNGLI VELDGKRRDQFDMIDRFIADYAINVERTEQAMRLEAVEIARMLVDIHVS REEIIAITTAITPAKAVEVMAQMNVVEMMMALQKMRARRTPSNQCHVTN LKDNPVQIAADAAEAGIRGFSEQETTVGIARYAPFNALALLVGSQCGRP GVLTQCSVEEATELELGMRGLTSYAETVSVYGTEAVFTDGDDTPWSKAF LASAYASRGLKMRYTSGTGSEALMGYSESKSMLYLESRCIFITKGAGVQ GLQNGAVSCIGMTGAVPSGIRAVLAENLIASMLDLEVASANDQTFSHSD IRRTARTLMQMLPGTDFIFSGYSAVPNYDNMFAGSNFDAEDFDDYNILQ RDLMVDGGLRPVTEAETIAIRQKAARAIQAVFRELGLPPIADEEVEAAT YAHGSNEMPPRNV**W**EDLSAVEEMMKRNITGLDIVGALSRSGFEDIASNI LNMLRQRVTGDYLQTSAILDRQFEVVSAVNDINDYQGPGTGYRISAERW AEIKNIPGVVQPDTIE**QGGIPV**QQTTQIQPSFTLKTREGGVASADERAD EVVIGVGPAFDKHQHHTLIDMPHGAILKELIAGVEEEGLHARVVRILRT SDVSFMAWDAANLSGSGIGIGIQSKGTTVIHQRDLLPLSNLELFSQAPL LTLETYRQIGKNAARYARKESPSPVPVVNDQMVRPKFMAKAALFHIKET KHVVQDAEPVTLHIDLVRE (SEQ ID NO.: 6) |

(continued)

| Variant | Amino acid sequence of α-β fusion subunit |
|---|---|
| (β)L113W | MKRSKRFAVLAQRPVNQDGLIGEWPEEGLIAMDSPFDPVSSVKVDNGLI VELDGKRRDQFDMIDRFIADYAINVERTEQAMRLEAVEIARMLVDIHVS REEIIAITTAITPAKAVEVMAQMNVVEMMMALQKMRARRTPSNQCHVTN LKDNPVQIAADAAEAGIRGFSEQETTVGIARYAPFNALALLVGSQCGRP GVLTQCSVEEATELELGMRGLTSYAETVSVYGTEAVFTDGDDTPWSKAF LASAYASRGLKMRYTSGTGSEALMGYSESKSMLYLESRCIFITKGAGVQ GLQNGAVSCIGMTGAVPSGIRAVLAENLIASMLDLEVASANDQTFSHSD IRRTARTLMQMLPGTDFIFSGYSAVPNYDNMFAGSNFDAEDFDDYNILQ RDLMVDGGLRPVTEAETIAIRQKAARAIQAVFRELGLPPIADEEVEAAT YAHGSNEMPPRNVVEDLSAVEEMMKRNITGLDIVGALSRSGFEDIASNI LNMLRQRVTGDYLQTSAILDRQFEVVSAVNDINDYQGPGTGYRISAERW AEIKNIPGVVQPDTIE**QGGIPV**QQTTQIQPSFTLKTREGGVASADERAD EVVIGVGPAFDKHQHHTLIDMPHGAILKELIAGVEEEGLHARVVRILRT SDVSFMAWDAANLSGSGIGIGIQSKGTTVIHQRDL**W**PLSNLELFSQAPL LTLETYRQIGKNAARYARKESPSPVPVVNDQMVRPKFMAKAALFHIKET KHVVQDAEPVTLHIDLVRE (SEQ ID NO.: 7) |
| (β)L113F | MKRSKRFAVLAQRPVNQDGLIGEWPEEGLIAMDSPFDPVSSVKVDNGLI VELDGKRRDQFDMIDRFIADYAINVERTEQAMRLEAVEIARMLVDIHVS REEIIAITTAITPAKAVEVMAQMNVVEMMMALQKMRARRTPSNQCHVTN LKDNPVQIAADAAEAGIRGFSEQETTVGIARYAPFNALALLVGSQCGRP GVLTQCSVEEATELELGMRGLTSYAETVSVYGTEAVFTDGDDTPWSKAF LASAYASRGLKMRYTSGTGSEALMGYSESKSMLYLESRCIFITKGAGVQ GLQNGAVSCIGMTGAVPSGIRAVLAENLIASMLDLEVASANDQTFSHSD IRRTARTLMQMLPGTDFIFSGYSAVPNYDNMFAGSNFDAEDFDDYNILQ RDLMVDGGLRPVTEAETIAIRQKAARAIQAVFRELGLPPIADEEVEAAT YAHGSNEMPPRNVVEDLSAVEEMMKRNITGLDIVGALSRSGFEDIASNI LNMLRQRVTGDYLQTSAILDRQFEVVSAVNDINDYQGPGTGYRISAERW AEIKNIPGVVQPDTIE**QGGIPV**QQTTQIQPSFTLKTREGGVASADERAD EVVIGVGPAFDKHQHHTLIDMPHGAILKELIAGVEEEGLHARVVRILRT SDVSFMAWDAANLSGSGIGIGIQSKGTTVIHQRDL**F**PLSNLELFSQAPL LTLETYRQIGKNAARYARKESPSPVPVVNDQMVRPKFMAKAALFHIKET KHVVQDAEPVTLHIDLVRE (SEQ ID NO.: 8) |
| (β) M158W | MKRSKRFAVLAQRPVNQDGLIGEWPEEGLIAMDSPFDPVSSVKVDNGLI VELDGKRRDQFDMIDRFIADYAINVERTEQAMRLEAVEIARMLVDIHVS REEIIAITTAITPAKAVEVMAQMNVVEMMMALQKMRARRTPSNQCHVTN LKDNPVQIAADAAEAGIRGFSEQETTVGIARYAPFNALALLVGSQCGRP GVLTQCSVEEATELELGMRGLTSYAETVSVYGTEAVFTDGDDTPWSKAF LASAYASRGLKMRYTSGTGSEALMGYSESKSMLYLESRCIFITKGAGVQ GLQNGAVSCIGMTGAVPSGIRAVLAENLIASMLDLEVASANDQTFSHSD IRRTARTLMQMLPGTDFIFSGYSAVPNYDNMFAGSNFDAEDFDDYNILQ RDLMVDGGLRPVTEAETIAIRQKAARAIQAVFRELGLPPIADEEVEAAT YAHGSNEMPPRNVVEDLSAVEEMMKRNITGLDIVGALSRSGFEDIASNI |

(continued)

| Variant | Amino acid sequence of α-β fusion subunit |
|---|---|
| | LNMLRQRVTGDYLQTSAILDRQFEVVSAVNDINDYQGPGTGYRISAERW AEIKNIPGVVQPDTIE**QGGIPV**QQTTQIQPSFTLKTREGGVASADERAD EVVIGVGPAFDKHQHHTLIDMPHGAILKELIAGVEEEGLHARVVRILRT SDVSFMAWDAANLSGSGIGIGIQSKGTTVIHQRDLLPLSNLELFSQAPL LTLETYRQIGKNAARYARKESPSPVPVVNDQ**W**VRPKFMAKAALFHIKET KHVVQDAEPVTLHIDLVRE (SEQ ID NO.: 9) |
| (α)A177M/ (β) L113W | MKRSKRFAVLAQRPVNQDGLIGEWPEEGLIAMDSPFDPVSSVKVDNGLI VELDGKRRDQFDMIDRFIADYAINVERTEQAMRLEAVEIARMLVDIHVS REEIIAITTAITPAKAVEVMAQMNVVEMMMALQKMRARRTPSNQCHVTN LKDNPVQIAADAAEAGIRGFSEQETTVGI**M**RYAPFNALALLVGSQCGRP GVLTQCSVEEATELELGMRGLTSYAETVSVYGTEAVFTDGDDTPWSKAF LASAYASRGLKMRYTSGTGSEALMGYSESKSMLYLESRCIFITKGAGVQ GLQNGAVSCIGMTGAVPSGIRAVLAENLIASMLDLEVASANDQTFSHSD IRRTARTLMQMLPGTDFIFSGYSAVPNYDNMFAGSNFDAEDFDDYNILQ RDLMVDGGLRPVTEAETIAIRQKAARAIQAVFRELGLPPIADEEVEAAT YAHGSNEMPPRNVVEDLSAVEEMMKRNITGLDIVGALSRSGFEDIASNI LNMLRQRVTGDYLQTSAILDRQFEVVSAVNDINDYQGPGTGYRISAERW AEIKNIPGVVQPDTIE**QGGIPV**QQTTQIQPSFTLKTREGGVASADERAD EVVIGVGPAFDKHQHHTLIDMPHGAILKELIAGVEEEGLHARVVRILRT SDVSFMAWDAANLSGSGIGIGIQSKGTTVIHQRDL**W**PLSNLELFSQAPL LTLETYRQIGKNAARYARKESPSPVPVVNDQMVRPKFMAKAALFHIKET KHVVQDAEPVTLHIDLVRE (SEQ ID NO.: 10) |
| (α)A177M/ (β) M158W | MKRSKRFAVLAQRPVNQDGLIGEWPEEGLIAMDSPFDPVSSVKVDNGLI VELDGKRRDQFDMIDRFIADYAINVERTEQAMRLEAVEIARMLVDIHVS REEIIAITTAITPAKAVEVMAQMNVVEMMMALQKMRARRTPSNQCHVTN LKDNPVQIAADAAEAGIRGFSEQETTVGI**M**RYAPFNALALLVGSQCGRP GVLTQCSVEEATELELGMRGLTSYAETVSVYGTEAVFTDGDDTPWSKAF LASAYASRGLKMRYTSGTGSEALMGYSESKSMLYLESRCIFITKGAGVQ GLQNGAVSCIGMTGAVPSGIRAVLAENLIASMLDLEVASANDQTFSHSD IRRTARTLMQMLPGTDFIFSGYSAVPNYDNMFAGSNFDAEDFDDYNILQ RDLMVDGGLRPVTEAETIAIRQKAARAIQAVFRELGLPPIADEEVEAAT YAHGSNEMPPRNVVEDLSAVEEMMKRNITGLDIVGALSRSGFEDIASNI LNMLRQRVTGDYLQTSAILDRQFEVVSAVNDINDYQGPGTGYRISAERW AEIKNIPGVVQPDTIE**QGGIPV**QQTTQIQPSFTLKTREGGVASADERAD EVVIGVGPAFDKHQHHTLIDMPHGAILKELIAGVEEEGLHARVVRILRT SDVSFMAWDAANLSGSGIGIGIQSKGTTVIHQRDLLPLSNLELFSQAPL LTLETYRQIGKNAARYARKESPSPVPVVNDQ**W**VRPKFMAKAALFHIKET KHVVQDAEPVTLHIDLVRE (SEQ ID NO.: 11) |

(continued)

| Variant | Amino acid sequence of α-β fusion subunit |
|---|---|
| (α)A177M/ (β) L113W/ (β) M158W | MKRSKRFAVLAQRPVNQDGLIGEWPEEGLIAMDSPFDPVSSVKVDNGLI VELDGKRRDQFDMIDRFIADYAINVERTEQAMRLEAVEIARMLVDIHVS REEIIAITTAITPAKAVEVMAQMNVVEMMMALQKMRARRTPSNQCHVTN LKDNPVQIAADAAEAGIRGFSEQETTVGI**M**RYAPFNALALLVGSQCGRP GVLTQCSVEEATELELGMRGLTSYAETVSVYGTEAVFTDGDDTPWSKAF LASAYASRGLKMRYTSGTGSEALMGYSESKSMLYLESRCIFITKGAGVQ GLQNGAVSCIGMTGAVPSGIRAVLAENLIASMLDLEVASANDQTFSHSD IRRTARTLMQMLPGTDFIFSGYSAVPNYDNMFAGSNFDAEDFDDYNILQ RDLMVDGGLRPVTEAETIAIRQKAARAIQAVFRELGLPPIADEEVEAAT YAHGSNEMPPRNVVEDLSAVEEMMKRNITGLDIVGALSRSGFEDIASNI LNMLRQRVTGDYLQTSAILDRQFEVVSAVNDINDYQGPGTGYRISAERW AEIKNIPGVVQPDTIE**QGGIPV**QQTTQIQPSFTLKTREGGVASADERAD EVVIGVGPAFDKHQHHTLIDMPHGAILKELIAGVEEEGLHARVVRILRT SDVSFMAWDAANLSGSGIGIGIQSKGTTVIHQRDL**W**PLSNLELFSQAPL LTLETYRQIGKNAARYARKESPSPVPVVNDQ**W**VRPKFMAKAALFHIKET KHVVQDAEPVTLHIDLVRE (SEQ ID NO.: 12) |

[Table 7]

| Variant | Base sequence of α-β fusion subunit |
|---------|-------------------------------------|
| (α)A177M | ATGAAAAGATCAAAACGATTTGCAGTACTGGCCCAGCGCCCCGTCAATCAGGA CGGGCTGATTGGCGAGTGGCCTGAAGAGGGGCTGATCGCCATGGACAGCCCCT TTGACCCGGTCTCTTCAGTAAAAGTGGACAACGGTCTGATCGTCGAGCTGGAC GGCAAACGCCGGGACCAGTTTGACATGATCGACCGATTTATCGCCGATTACGC GATCAACGTTGAGCGCACAGAGCAGGCAATGCGCCTGGAGGCGGTGGAAATAG CCCGCATGCTGGTGGATATTCACGTCAGTCGGGAGGAGATCATTGCCATCACT ACCGCCATCACGCCGGCCAAAGCGGTCGAGGTGATGGCGCAGATGAACGTGGT GGAGATGATGATGGCGCTGCAGAAGATGCGTGCCCGCCGGACCCCCTCCAACC AGTGCCACGTCACCAATCTCAAAGATAATCCGGTGCAGATTGCTGCTGACGCC GCCGAGGCCGGGATCCGCGGCTTCTCAGAACAGGAGACCACGGTCGGTATC**AT** GCGCTATGCGCCGTTTAACGCCCTGGCGCTGTTGGTCGGTTCGCAGTGCGGCC GCCCCGGCGTTTTGACGCAGTGCTCGGTGGAAGAGGCCACCGAGCTGGAGCTG GGCATGCGTGGCTTAACCAGCTACGCCGAGACGGTGTCGGTCTACGGCACCGA AGCGGTATTTACCGACGGCGATGATACTCCGTGGTCAAAGGCGTTCCTCGCCT CGGCCTACGCCTCCCGCGGGTTGAAAATGCGCTACACCTCCGGCACCGGATCC GAAGCGCTGATGGGCTATTCGGAGAGCAAGTCGATGCTCTACCTCGAATCGCG CTGCATCTTCATTACCAAAGGCGCCGGGGTTCAGGGGCTGCAAAACGGCGCGG TGAGCTGTATCGGCATGACCGGCGCTGTGCCGTCGGGCATTCGGGCGGTGCTG GCGGAAAACCTGATCGCCTCTATGCTCGACCTCGAAGTGGCGTCCGCCAACGA CCAGACTTTCTCCCACTCGGATATTCGCCGCACCGCGCGCACCCTGATGCAGA TGCTGCCGGGCACCGACTTTATTTTCTCCGGCTACAGCGCGGTGCCGAACTAC GACAACATGTTCGCCGGCTCGAACTTCGATGCGGAAGATTTTGATGATTACAA CATCCTGCAGCGTGACCTGATGGTTGACGGCGGCCTGCGTCCGGTGACCGAGG CGGAAACCATTGCCATTCGCCAGAAAGCGGCGCGGGCGATCCAGGCGGTTTTC CGCGAGCTGGGGCTGCCGCCAATCGCCGACGAGGAGGTGGAGGCCGCCACCTA CGCGCACGGTAGCAACGAGATGCCGCCGCGTAACGTGGTGGAGGATCTGAGTG CGGTGGAAGAGATGATGAAGCGCAACATCACCGGCCTCGATATTGTCGGCGCG CTGAGCCGCAGCGGCTTTGAGGATATCGCCAGCAATATTCTCAATATGCTGCG CCAGCGGGTCACCGGCGATTACCTGCAGACCTCGGCCATTCTCGATCGGCAGT TCGAGGTGGTGAGTGCGGTCAACGACATCAATGACTATCAGGGGCCGGGCACC GGCTATCGCATCTCTGCCGAACGCTGGGCGGAGATCAAAAATATTCCGGGCGT GGTTCAGCCCGACACCATTGAA**C**AAGGCGGTATTCCTGTGCAACAGACAACCC AAATTCAGCCCTCTTTTACCCTGAAAACCCGCGAGGGCGGGGTAGCTTCTGCC GATGAACGCGCCGATGAAGTGGTGATCGGCGTCGGCCCTGCCTTCGATAAACA CCAGCATCACACTCTGATCGATATGCCCCATGGCGCGATCCTCAAAGAGCTGA TTGCCGGGGTGGAAGAAGAGGGGCTTCACGCCCGGGTGGTGCGCATTCTGCGC ACGTCCGACGTCTCCTTTATGGCCTGGGATGCGGCCAACCTGAGCGGCTCGGG GATCGGCATCGGTATCCAGTCGAAGGGGACCACGGTCATCCATCAGCGCGATC TGCTGCCGCTCAGCAACCTGGAGCTGTTCTCCCAGGCGCCGCTGCTGACGCTG GAAACCTACCGGCAGATTGGCAAAAACGCCGCGCGCTATGCGCGCAAAGAGTC ACCTTCGCCGGTGCCGGTGGTGAACGATCAGATGGTGCGGCCGAAATTTATGG CCAAAGCCGCGCTATTTCATATCAAAGAGACCAAACATGTGGTGCAGGACGCC GAGCCCGTCACCCTGCACGTCGACTTAGTAAGGGAGTGA (SEQ ID NO.: 17) |

(continued)

| Variant | Base sequence of α-β fusion subunit |
|---|---|
| (α)V455W | ATGAAAAGATCAAAACGATTTGCAGTACTGGCCCAGCGCCCCGTCAATCAGGACGGGCTGATTGGCGAGTGGCCTGAAGAGGGGCTGATCGCCATGGACAGCCCCTTTGACCCGGTCTCTTCAGTAAAAGTGGACAACGGTCTGATCGTCGAGCTGGACGGCAAACGCCGGGACCAGTTTGACATGATCGACCGATTTATCGCCGATTACGCGATCAACGTTGAGCGCACAGAGCAGGCAATGCGCCTGGAGGCGGTGGAAATAGCCCGCATGCTGGTGGATATTCACGTCAGTCGGGAGGAGATCATTGCCATCACTACCGCCATCACGCCGGCCAAAGCGGTCGAGGTGATGGCGCAGATGAACGTGGTGGAGATGATGATGGCGCTGCAGAAGATGCGTGCCCGCCGGACCCCCTCCAACCAGTGCCACGTCACCAATCTCAAAGATAATCCGGTGCAGATTGCTGCTGACGCCGCCGAGGCCGGGATCCGCGGCTTCTCAGAACAGGAGACCACGGTCGGTATCGCGCGCTATGCGCCGTTTAACGCCCTGGCGCTGTTGGTCGGTTCGCAGTGCGGCCGCCCCGGCGTTTTGACGCAGTGCTCGGTGGAAGAGGCCACCGAGCTGGAGCTGGGCATGCGTGGCTTAACCAGCTACGCCGAGACGGTGTCGGTCTACGGCACCGAAGCGGTATTTACCGACGGCGATGATACTCCGTGGTCAAAGGCGTTCCTCGCCTCGGCCTACGCCTCCCGCGGGTTGAAAATGCGCTACACCTCCGGCACCGGATCCGAAGCGCTGATGGGCTATTCGGAGAGCAAGTCGATGCTCTACCTCGAATCGCGCTGCATCTTCATTACCAAAGGCGCCGGGGTTCAGGGGCTGCAAAACGGCGCGGTGAGCTGTATCGGCATGACCGGCGCTGTGCCGTCGGGCATTCGGGCGGTGCTGGCGGAAAACCTGATCGCCTCTATGCTCGACCTCGAAGTGGCGTCCGCCAACGACCAGACTTTCTCCCACTCGGATATTCGCCGCACCGCGCGCACCCTGATGCAGATGCTGCCGGGCACCGACTTTATTTTCTCCGGCTACAGCGCGGTGCCGAACTACGACAACATGTTCGCCGGCTCGAACTTCGATGCGGAAGATTTTGATGATTACAACATCCTGCAGCGTGACCTGATGGTTGACGGCGGCCTGCGTCCGGTGACCGAGGCGGAAACCATTGCCATTCGCCAGAAAGCGGCGCGGGCGATCCAGGCGGTTTTCCGCGAGCTGGGGCTGCCGCCAATCGCCGACGAGGAGGTGGAGGCCGCCACCTACGCGCACGGTAGCAACGAGATGCCGCCGCGTAACGTG**TGG**GAGGATCTGAGTGCGGTGGAAGAGATGATGAAGCGCAACATCACCGGCCTCGATATTGTCGGCGCGCTGAGCCGCAGCGGCTTTGAGGATATCGCCAGCAATATTCTCAATATGCTGCGCCAGCGGGTCACCGGCGATTACCTGCAGACCTCGGCCATTCTCGATCGGCAGTTCGAGGTGGTGAGTGCGGTCAACGACATCAATGACTATCAGGGGCCGGGCACCGGCTATGCATCTCTGCCGAACGCTGGGCGGAGATCAAAAATATTCCGGGCGTGGTTCAGCCCGACACCATTGAA**C**AAGGCGGTATTCCTGTGCAACAGACAACCCAAATTCAGCCCTCTTTTACCCTGAAAACCCGCGAGGGCGGGGTAGCTTCTGCCGATGAACGCGCCGATGAAGTGGTGATCGGCGTCGGCCCTGCCTTCGATAAACACCAGCATCACACTCTGATCGATATGCCCCATGGCGCGATCCTCAAAGAGCTGATTGCCGGGGTGGAAGAAGAGGGGCTTCACGCCCGGGTGGTGCGCATTCTGCGCACGTCCGACGTCTCCTTTATGGCCTGGGATGCGGCCAACCTGAGCGGCTCGGGGATCGGCATCGGTATCCAGTCGAAGGGGACCACGGTCATCCATCAGCGCGATCTGCTGCCGCTCAGCAACCTGGAGCTGTTCTCCCAGGCGCCGCTGCTGACGCTGGAAACCTACCGGCAGATTGGCAAAAACGCCGCGCGCTATGCGCGCAAAGAGTCACCTTCGCCGGTGCCGGTGGTGAACGATCAGATGGTGCGGCCGAAATTTATGGCCAAAGCCGCGCTATTTCATATCAAAGAGACCAAACATGTGGTGCAGGACGCCGAGCCCGTCACCCTGCACGTCGACTTAGTAAGGGAGTGA (SEQ ID NO.: 18) |

(continued)

| Variant | Base sequence of α-β fusion subunit |
|---|---|
| (β)L113W | ATGAAAAGATCAAAACGATTTGCAGTACTGGCCCAGCGCCCCGTCAATCAGGA<br>CGGGCTGATTGGCGAGTGGCCTGAAGAGGGGCTGATCGCCATGGACAGCCCCT<br>TTGACCCGGTCTCTTCAGTAAAAGTGGACAACGGTCTGATCGTCGAGCTGGAC<br>GGCAAACGCCGGGACCAGTTTGACATGATCGACCGATTTATCGCCGATTACGC<br>GATCAACGTTGAGCGCACAGAGCAGGCAATGCGCCTGGAGGCGGTGGAAATAG<br>CCCGCATGCTGGTGGATATTCACGTCAGTCGGGAGGAGATCATTGCCATCACT<br>ACCGCCATCACGCCGGCCAAAGCGGTCGAGGTGATGGCGCAGATGAACGTGGT<br>GGAGATGATGATGGCGCTGCAGAAGATGCGTGCCCGCCGGACCCCCTCCAACC<br>AGTGCCACGTCACCAATCTCAAAGATAATCCGGTGCAGATTGCTGCTGACGCC<br>GCCGAGGCCGGGATCCGCGGCTTCTCAGAACAGGAGACCACGGTCGGTATCGC<br>GCGCTATGCGCCGTTTAACGCCCTGGCGCTGTTGGTCGGTTCGCAGTGCGGCC<br>GCCCCGGCGTTTTGACGCAGTGCTCGGTGGAAGAGGCCACCGAGCTGGAGCTG<br>GGCATGCGTGGCTTAACCAGCTACGCCGAGACGGTGTCGGTCTACGGCACCGA<br>AGCGGTATTTACCGACGGCGATGATACTCCGTGGTCAAAGGCGTTCCTCGCCT<br>CGGCCTACGCCTCCCGCGGGTTGAAAATGCGCTACACCTCCGGCACCGGATCC<br>GAAGCGCTGATGGGCTATTCGGAGAGCAAGTCGATGCTCTACCTCGAATCGCG<br>CTGCATCTTCATTACCAAAGGCGCCGGGGTTCAGGGGCTGCAAAACGGCGCGG<br>TGAGCTGTATCGGCATGACCGGCGCTGTGCCGTCGGGCATTCGGGCGGTGCTG<br>GCGGAAAACCTGATCGCCTCTATGCTCGACCTCGAAGTGGCGTCCGCCAACGA<br>CCAGACTTTCTCCCACTCGGATATTCGCCGCACCGCGCGCACCCTGATGCAGA<br>TGCTGCCGGGCACCGACTTTATTTTCTCCGGCTACAGCGCGGTGCCGAACTAC<br>GACAACATGTTCGCCGGCTCGAACTTCGATGCGGAAGATTTTGATGATTACAA<br>CATCCTGCAGCGTGACCTGATGGTTGACGGCGGCCTGCGTCCGGTGACCGAGG<br>CGGAAACCATTGCCATTCGCCAGAAAGCGGCGCGGGCGATCCAGGCGGTTTTC<br>CGCGAGCTGGGGCTGCCGCCAATCGCCGACGAGGAGGTGGAGGCCGCCACCTA<br>CGCGCACGGTAGCAACGAGATGCCGCCGCGTAACGTGGTGGAGGATCTGAGTG<br>CGGTGGAAGAGATGATGAAGCGCAACATCACCGGCCTCGATATTGTCGGCGCG<br>CTGAGCCGCAGCGGCTTTGAGGATATCGCCAGCAATATTCTCAATATGCTGCG<br>CCAGCGGGTCACCGGCGATTACCTGCAGACCTCGGCCATTCTCGATCGGCAGT<br>TCGAGGTGGTGAGTGCGGTCAACGACATCAATGACTATCAGGGGCCGGGCACC<br>GGCTATCGCATCTCTGCCGAACGCTGGGCGGAGATCAAAAATATTCCGGGCGT<br>GGTTCAGCCCGACACCATTGAA**<u>C</u>**AAGGCGGTATTCCTGTGCAACAGACAACCC<br>AAATTCAGCCCTCTTTTACCCTGAAAACCCGCGAGGGCGGGGTAGCTTCTGCC<br>GATGAACGCGCCGATGAAGTGGTGATCGGCGTCGGCCCTGCCTTCGATAAACA<br>CCAGCATCACACTCTGATCGATATGCCCCATGGCGCGATCCTCAAAGAGCTGA<br>TTGCCGGGGTGGAAGAAGAGGGGCTTCACGCCCGGGTGGTGCGCATTCTGCGC<br>ACGTCCGACGTCTCCTTTATGGCCTGGGATGCGGCCAACCTGAGCGGCTCGGG<br>GATCGGCATCGGTATCCAGTCGAAGGGGACCACGGTCATCCATCAGCGCGATC<br>TG**<u>TG</u>**GCCGCTCAGCAACCTGGAGCTGTTCTCCCAGGCGCCGCTGCTGACGCTG<br>GAAACCTACCGGCAGATTGGCAAAAACGCCGCGCGCTATGCGCGCAAAGAGTC<br>ACCTTCGCCGGTGCCGGTGGTGAACGATCAGATGGTGCGGCCGAAATTTATGG<br>CCAAAGCCGCGCTATTTCATATCAAAGAGACCAAACATGTGGTGCAGGACGCC<br>GAGCCCGTCACCCTGCACGTCGACTTAGTAAGGGAGTGA (SEQ ID NO.:<br>19) |
| (β)L113F | ATGAAAAGATCAAAACGATTTGCAGTACTGGCCCAGCGCCCCGTCAATCAGGA<br>CGGGCTGATTGGCGAGTGGCCTGAAGAGGGGCTGATCGCCATGGACAGCCCCT<br>TTGACCCGGTCTCTTCAGTAAAAGTGGACAACGGTCTGATCGTCGAGCTGGAC<br>GGCAAACGCCGGGACCAGTTTGACATGATCGACCGATTTATCGCCGATTACGC |

(continued)

| Variant | Base sequence of α-β fusion subunit |
|---|---|
| | GATCAACGTTGAGCGCACAGAGCAGGCAATGCGCCTGGAGGCGGTGGAAATAG<br>CCCGCATGCTGGTGGATATTCACGTCAGTCGGGAGGAGATCATTGCCATCACT<br>ACCGCCATCACGCCGGCCAAAGCGGTCGAGGTGATGGCGCAGATGAACGTGGT<br>GGAGATGATGATGGCGCTGCAGAAGATGCGTGCCCGCCGGACCCCCTCCAACC<br>AGTGCCACGTCACCAATCTCAAAGATAATCCGGTGCAGATTGCTGCTGACGCC<br>GCCGAGGCCGGGATCCGCGGCTTCTCAGAACAGGAGACCACGGTCGGTATCGC<br>GCGCTATGCGCCGTTTAACGCCCTGGCGCTGTTGGTCGGTTCGCAGTGCGGCC<br>GCCCCGGCGTTTTGACGCAGTGCTCGGTGGAAGAGGCCACCGAGCTGGAGCTG<br>GGCATGCGTGGCTTAACCAGCTACGCCGAGACGGTGTCGGTCTACGGCACCGA<br>AGCGGTATTTACCGACGGCGATGATACTCCGTGGTCAAAGGCGTTCCTCGCCT<br>CGGCCTACGCCTCCCGCGGGTTGAAAATGCGCTACACCTCCGGCACCGGATCC<br>GAAGCGCTGATGGGCTATTCGGAGAGCAAGTCGATGCTCTACCTCGAATCGCG<br>CTGCATCTTCATTACCAAAGGCGCCGGGGTTCAGGGGCTGCAAAACGGCGCGG<br>TGAGCTGTATCGGCATGACCGGCGCTGTGCCGTCGGGCATTCGGGCGGTGCTG<br>GCGGAAAACCTGATCGCCTCTATGCTCGACCTCGAAGTGGCGTCCGCCAACGA<br>CCAGACTTTCTCCCACTCGGATATTCGCCGCACCGCGCGCACCCTGATGCAGA<br>TGCTGCCGGGCACCGACTTTATTTTCTCCGGCTACAGCGCGGTGCCGAACTAC<br>GACAACATGTTCGCCGGCTCGAACTTCGATGCGGAAGATTTTGATGATTACAA<br>CATCCTGCAGCGTGACCTGATGGTTGACGGCGGCCTGCGTCCGGTGACCGAGG<br>CGGAAACCATTGCCATTCGCCAGAAAGCGGCGCGGGCGATCCAGGCGGTTTTC<br>CGCGAGCTGGGGCTGCCGCCAATCGCCGACGAGGAGGTGGAGGCCGCCACCTA<br>CGCGCACGGTAGCAACGAGATGCCGCCGCGTAACGTGGTGGAGGATCTGAGTG<br>CGGTGGAAGAGATGATGAAGCGCAACATCACCGGCCTCGATATTGTCGGCGCG<br>CTGAGCCGCAGCGGCTTTGAGGATATCGCCAGCAATATTCTCAATATGCTGCG<br>CCAGCGGGTCACCGGCGATTACCTGCAGACCTCGGCCATTCTCGATCGGCAGT<br>TCGAGGTGGTGAGTGCGGTCAACGACATCAATGACTATCAGGGGCCGGGCACC<br>GGCTATCGCATCTCTGCCGAACGCTGGGCGGAGATCAAAAATATTCCGGGCGT<br>GGTTCAGCCCGACACCATTGAA<u>**C**</u>AAGGCGGTATTCCTGTGCAACAGACAACCC<br>AAATTCAGCCCTCTTTTACCCTGAAAACCCGCGAGGGCGGGGTAGCTTCTGCC<br>GATGAACGCGCCGATGAAGTGGTGATCGGCGTCGGCCCTGCCTTCGATAAACA<br>CCAGCATCACACTCTGATCGATATGCCCCATGGCGCGATCCTCAAAGAGCTGA<br>TTGCCGGGGTGGAAGAAGAGGGGCTTCACGCCCGGGTGGTGCGCATTCTGCGC<br>ACGTCCGACGTCTCCTTTATGGCCTGGGATGCGGCCAACCTGAGCGGCTCGGG<br>GATCGGCATCGGTATCCAGTCGAAGGGGACCACGGTCATCCATCAGCGCGATC<br>TG<u>**TTT**</u>CCGCTCAGCAACCTGGAGCTGTTCTCCCAGGCGCCGCTGCTGACGCTG<br>GAAACCTACCGGCAGATTGGCAAAAACGCCGCGCGCTATGCGCGCAAAGAGTC<br>ACCTTCGCCGGTGCCGGTGGTGAACGATCAGATGGTGCGGCCGAAATTTATGG<br>CCAAAGCCGCGCTATTTCATATCAAAGAGACCAAACATGTGGTGCAGGACGCC<br>GAGCCCGTCACCCTGCACGTCGACTTAGTAAGGGAGTGA (SEQ ID NO.:<br>20) |

(continued)

| Variant | Base sequence of α-β fusion subunit |
|---------|-------------------------------------|
| (β) M158W | ATGAAAAGATCAAAACGATTTGCAGTACTGGCCCAGCGCCCCGTCAATCAGGA CGGGCTGATTGGCGAGTGGCCTGAAGAGGGGCTGATCGCCATGGACAGCCCCT TTGACCCGGTCTCTTCAGTAAAAGTGGACAACGGTCTGATCGTCGAGCTGGAC GGCAAACGCCGGGACCAGTTTGACATGATCGACCGATTTATCGCCGATTACGC GATCAACGTTGAGCGCACAGAGCAGGCAATGCGCCTGGAGGCGGTGGAAATAG CCCGCATGCTGGTGGATATTCACGTCAGTCGGGAGGAGATCATTGCCATCACT ACCGCCATCACGCCGGCCAAAGCGGTCGAGGTGATGGCGCAGATGAACGTGGT GGAGATGATGATGGCGCTGCAGAAGATGCGTGCCCGCCGGACCCCCTCCAACC AGTGCCACGTCACCAATCTCAAAGATAATCCGGTGCAGATTGCTGCTGACGCC GCCGAGGCCGGGATCCGCGGCTTCTCAGAACAGGAGACCACGGTCGGTATCGC GCGCTATGCGCCGTTTAACGCCCTGGCGCTGTTGGTCGGTTCGCAGTGCGGCC GCCCCGGCGTTTTGACGCAGTGCTCGGTGGAAGAGGCCACCGAGCTGGAGCTG |
| | GGCATGCGTGGCTTAACCAGCTACGCCGAGACGGTGTCGGTCTACGGCACCGA AGCGGTATTTACCGACGGCGATGATACTCCGTGGTCAAAGGCGTTCCTCGCCT CGGCCTACGCCTCCCGCGGGTTGAAAATGCGCTACACCTCCGGCACCGGATCC GAAGCGCTGATGGGCTATTCGGAGAGCAAGTCGATGCTCTACCTCGAATCGCG CTGCATCTTCATTACCAAAGGCGCCGGGGTTCAGGGGCTGCAAAACGGCGCGG TGAGCTGTATCGGCATGACCGGCGCTGTGCCGTCGGGCATTCGGGCGGTGCTG GCGGAAAACCTGATCGCCTCTATGCTCGACCTCGAAGTGGCGTCCGCCAACGA CCAGACTTTCTCCCACTCGGATATTCGCCGCACCGCGCGCACCCTGATGCAGA TGCTGCCGGGCACCGACTTTATTTTCTCCGGCTACAGCGCGGTGCCGAACTAC GACAACATGTTCGCCGGCTCGAACTTCGATGCGGAAGATTTTGATGATTACAA CATCCTGCAGCGTGACCTGATGGTTGACGGCGGCCTGCGTCCGGTGACCGAGG CGGAAACCATTGCCATTCGCCAGAAAGCGGCGCGGGCGATCCAGGCGGTTTTC CGCGAGCTGGGGCTGCCGCCAATCGCCGACGAGGAGGTGGAGGCCGCCACCTA CGCGCACGGTAGCAACGAGATGCCGCCGCGTAACGTG**TGG**GAGGATCTGAGTG CGGTGGAAGAGATGATGAAGCGCAACATCACCGGCCTCGATATTGTCGGCGCG CTGAGCCGCAGCGGCTTTGAGGATATCGCCAGCAATATTCTCAATATGCTGCG CCAGCGGGTCACCGGCGATTACCTGCAGACCTCGGCCATTCTCGATCGGCAGT TCGAGGTGGTGAGTGCGGTCAACGACATCAATGACTATCAGGGGCCGGGCACC GGCTATCGCATCTCTGCCGAACGCTGGGCGGAGATCAAAAATATTCCGGGCGT GGTTCAGCCCGACACCATTGAA**C**AAGGCGGTATTCCTGTGCAACAGACAACCC AAATTCAGCCCTCTTTTACCCTGAAAACCCGCGAGGGCGGGGTAGCTTCTGCC GATGAACGCGCCGATGAAGTGGTGATCGGCGTCGGCCCTGCCTTCGATAAACA CCAGCATCACACTCTGATCGATATGCCCCATGGCGCGATCCTCAAAGAGCTGA TTGCCGGGGTGGAAGAAGAGGGGCTTCACGCCCGGGTGGTGCGCATTCTGCGC ACGTCCGACGTCTCCTTTATGGCCTGGGATGCGGCCAACCTGAGCGGCTCGGG GATCGGCATCGGTATCCAGTCGAAGGGGACCACGGTCATCCATCAGCGCGATC TGCTGCCGCTCAGCAACCTGGAGCTGTTCTCCCAGGCGCCGCTGCTGACGCTG GAAACCTACCGGCAGATTGGCAAAAACGCCGCGCGCTATGCGCGCAAAGAGTC ACCTTCGCCGGTGCCGGTGGTGAACGATCAGATGGTGCGGCCGAAATTTATGG CCAAAGCCGCGCTATTTCATATCAAAGAGACCAAACATGTGGTGCAGGACGCC GAGCCCGTCACCCTGCACGTCGACTTAGTAAGGGAGTGA (SEQ ID NO.: 21) |

(continued)

| Variant | Base sequence of α-β fusion subunit |
|---|---|
| (α)A177M/ (β) L113W | ATGAAAAGATCAAAACGATTTGCAGTACTGGCCCAGCGCCCCGTCAATCAGGACGGGCTGATTGGCGAGTGGCCTGAAGAGGGGCTGATCGCCATGGACAGCCCCTTTGACCCGGTCTCTTCAGTAAAAGTGGACAACGGTCTGATCGTCGAGCTGGACGGCAAACGCCGGGACCAGTTTGACATGATCGACCGATTTATCGCCGATTACGCGATCAACGTTGAGCGCACAGAGCAGGCAATGCGCCTGGAGGCGGTGGAAATAGCCCGCATGCTGGTGGATATTCACGTCAGTCGGGAGGAGATCATTGCCATCACTACCGCCATCACGCCGGCCAAAGCGGTCGAGGTGATGGCGCAGATGAACGTGGTGGAGATGATGATGGCGCTGCAGAAGATGCGTGCCCGCCGGACCCCCTCCAACCAGTGCCACGTCACCAATCTCAAAGATAATCCGGTGCAGATTGCTGCTGACGCCGCCGAGGCCGGGATCCGCGGCTTCTCAGAACAGGAGACCACGGTCGGTATC**AT**GCGCTATGCGCCGTTTAACGCCCTGGCGCTGTTGGTCGGTTCGCAGTGCGGCCGCCCCGGCGTTTTGACGCAGTGCTCGGTGGAAGAGGCCACCGAGCTGGAGCTGGGCATGCGTGGCTTAACCAGCTACGCCGAGACGGTGTCGGTCTACGGCACCGAAGCGGTATTTACCGACGGCGATGATACTCCGTGGTCAAAGGCGTTCCTCGCCTCGGCCTACGCCTCCCGCGGGTTGAAAATGCGCTACACCTCCGGCACCGGATCCGAAGCGCTGATGGGCTATTCGGAGAGCAAGTCGATGCTCTACCTCGAATCGCGCTGCATCTTCATTACCAAAGGCGCCGGGGTTCAGGGGCTGCAAAACGGCGCGGTGAGCTGTATCGGCATGACCGGCGCTGTGCCGTCGGGCATTCGGGCGGTGCTGGCGGAAAACCTGATCGCCTCTATGCTCGACCTCGAAGTGGCGTCCGCCAACGACCAGACTTTCTCCCACTCGGATATTCGCCGCACCGCGCGCACCCTGATGCAGA

TGCTGCCGGGCACCGACTTTATTTTCTCCGGCTACAGCGCGGTGCCGAACTACGACAACATGTTCGCCGGCTCGAACTTCGATGCGGAAGATTTTGATGATTACAACATCCTGCAGCGTGACCTGATGGTTGACGGCGGCCTGCGTCCGGTGACCGAGGCGGAAACCATTGCCATTCGCCAGAAAGCGGCGCGGGCGATCCAGGCGGTTTTCGCGAGCTGGGGCTGCCGCCAATCGCCGACGAGGAGGTGGAGGCCGCCACCTACGCGCACGGTAGCAACGAGATGCCGCCGCGTAACGTGGTGGAGGATCTGAGTGCGGTGGAAGAGATGATGAAGCGCAACATCACCGGCCTCGATATTGTCGGCGCGCTGAGCCGCAGCGGCTTTGAGGATATCGCCAGCAATATTCTCAATATGCTGCGCCAGCGGGTCACCGGCGATTACCTGCAGACCTCGGCCATTCTCGATCGGCAGTTCGAGGTGGTGAGTGCGGTCAACGACATCAATGACTATCAGGGGCCGGGCACCGGCTATCGCATCTCTGCCGAACGCTGGGCGGAGATCAAAAATATTCCGGGCGTGGTTCAGCCCGACACCATTGAA**C**AAGGCGGTATTCCTGTGCAACAGACAACCCAAATTCAGCCCTCTTTTACCCTGAAAACCCGCGAGGGCGGGGTAGCTTCTGCCGATGAACGCGCCGATGAAGTGGTGATCGGCGTCGGCCCTGCCTTCGATAAACACCAGCATCACACTCTGATCGATATGCCCCATGGCGCGATCCTCAAAGAGCTGATTGCCGGGGTGGAAGAAGAGGGGCTTCACGCCCGGGTGGTGCGCATTCTGCGCACGTCCGACGTCTCCTTTATGGCCTGGGATGCGGCCAACCTGAGCGGCTCGGGGATCGGCATCGGTATCCAGTCGAAGGGGACCACGGTCATCCATCAGCGCGATCTG**TG**GCCGCTCAGCAACCTGGAGCTGTTCTCCCAGGCGCCGCTGCTGACGCTGGAAACCTACCGGCAGATTGGCAAAAACGCCGCGCGCTATGCGCGCAAAGAGTCACCTTCGCCGGTGCCGGTGGTGAACGATCAGATGGTGCGGCCGAAATTTATGGCCAAAGCCGCGCTATTTCATATCAAAGAGACCAAACATGTGGTGCAGGACGCCGAGCCCGTCACCCTGCACGTCGACTTAGTAAGGGAGTGA (SEQ ID NO.: 22) |

(continued)

| Variant | Base sequence of α-β fusion subunit |
|---|---|
| (α)A177M/ (β) M158W | ATGAAAAGATCAAAACGATTTGCAGTACTGGCCCAGCGCCCCGTCAATCAGGA<br>CGGGCTGATTGGCGAGTGGCCTGAAGAGGGGCTGATCGCCATGGACAGCCCCT<br>TTGACCCGGTCTCTTCAGTAAAAGTGGACAACGGTCTGATCGTCGAGCTGGAC<br>GGCAAACGCCGGGACCAGTTTGACATGATCGACCGATTTATCGCCGATTACGC<br>GATCAACGTTGAGCGCACAGAGCAGGCAATGCGCCTGGAGGCGGTGGAAATAG<br>CCCGCATGCTGGTGGATATTCACGTCAGTCGGGAGGAGATCATTGCCATCACT<br>ACCGCCATCACGCCGGCCAAAGCGGTCGAGGTGATGGCGCAGATGAACGTGGT<br>GGAGATGATGATGGCGCTGCAGAAGATGCGTGCCCGCCGGACCCCCTCCAACC<br>AGTGCCACGTCACCAATCTCAAAGATAATCCGGTGCAGATTGCTGCTGACGCC<br>GCCGAGGCCGGGATCCGCGGCTTCTCAGAACAGGAGACCACGGTCGGTATC**AT**<br>GCGCTATGCGCCGTTTAACGCCCTGGCGCTGTTGGTCGGTTCGCAGTGCGGCC<br>GCCCCGGCGTTTTGACGCAGTGCTCGGTGGAAGAGGCCACCGAGCTGGAGCTG<br>GGCATGCGTGGCTTAACCAGCTACGCCGAGACGGTGTCGGTCTACGGCACCGA<br>AGCGGTATTTACCGACGGCGATGATACTCCGTGGTCAAAGGCGTTCCTCGCCT<br>CGGCCTACGCCTCCCGCGGGTTGAAAATGCGCTACACCTCCGGCACCGGATCC<br>GAAGCGCTGATGGGCTATTCGGAGAGCAAGTCGATGCTCTACCTCGAATCGCG<br>CTGCATCTTCATTACCAAAGGCGCCGGGGTTCAGGGGCTGCAAAACGGCGCGG<br>TGAGCTGTATCGGCATGACCGGCGCTGTGCCGTCGGGCATTCGGGCGGTGCTG<br>GCGGAAAACCTGATCGCCTCTATGCTCGACCTCGAAGTGGCGTCCGCCAACGA<br>CCAGACTTTCTCCCACTCGGATATTCGCCGCACCGCGCGCACCCTGATGCAGA<br>TGCTGCCGGGCACCGACTTTATTTTCTCCGGCTACAGCGCGGTGCCGAACTAC<br>GACAACATGTTCGCCGGCTCGAACTTCGATGCGGAAGATTTTGATGATTACAA<br>CATCCTGCAGCGTGACCTGATGGTTGACGGCGGCCTGCGTCCGGTGACCGAGG<br>CGGAAACCATTGCCATTCGCCAGAAAGCGGCGCGGGCGATCCAGGCGGTTTTC<br>CGCGAGCTGGGGCTGCCGCCAATCGCCGACGAGGAGGTGGAGGCCGCCACCTA<br>CGCGCACGGTAGCAACGAGATGCCGCCGCGTAACGTGGTGGAGGATCTGAGTG<br>CGGTGGAAGAGATGATGAAGCGCAACATCACCGGCCTCGATATTGTCGGCGCG<br>CTGAGCCGCAGCGGCTTTGAGGATATCGCCAGCAATATTCTCAATATGCTGCG |
| | CCAGCGGGTCACCGGCGATTACCTGCAGACCTCGGCCATTCTCGATCGGCAGT<br>TCGAGGTGGTGAGTGCGGTCAACGACATCAATGACTATCAGGGGCCGGGCACC<br>GGCTATCGCATCTCTGCCGAACGCTGGGCGGAGATCAAAAATATTCCGGGCGT<br>GGTTCAGCCCGACACCATTGAA**C**AAGGCGGTATTCCTGTGCAACAGACAACCC<br>AAATTCAGCCCTCTTTTACCCTGAAAACCCGCGAGGGCGGGGTAGCTTCTGCC<br>GATGAACGCGCCGATGAAGTGGTGATCGGCGTCGGCCCTGCCTTCGATAAACA<br>CCAGCATCACACTCTGATCGATATGCCCCATGGCGCGATCCTCAAAGAGCTGA<br>TTGCCGGGGTGGAAGAAGAGGGGCTTCACGCCCGGGTGGTGCGCATTCTGCGC<br>ACGTCCGACGTCTCCTTTATGGCCTGGGATGCGGCCAACCTGAGCGGCTCGGG<br>GATCGGCATCGGTATCCAGTCGAAGGGGACCACGGTCATCCATCAGCGCGATC<br>TGCTGCCGCTCAGCAACCTGGAGCTGTTCTCCCAGGCGCCGCTGCTGACGCTG<br>GAAACCTACCGGCAGATTGGCAAAAACGCCGCGCGCTATGCGCGCAAAGAGTC<br>ACCTTCGCCGGTGCCGGTGGTGAACGATCAG**TGG**GTGCGGCCGAAATTTATGG<br>CCAAAGCCGCGCTATTTCATATCAAAGAGACCAAACATGTGGTGCAGGACGCC<br>GAGCCCGTCACCCTGCACGTCGACTTAGTAAGGGAGTGA  (SEQ  ID  NO.:<br>23) |

(continued)

| Variant | Base sequence of α-β fusion subunit |
|---|---|
| (α)A177M/ (β) L113W/ (β) M158W | ATGAAAAGATCAAAACGATTTGCAGTACTGGCCCAGCGCCCCGTCAATCAGGA CGGGCTGATTGGCGAGTGGCCTGAAGAGGGGCTGATCGCCATGGACAGCCCCT TTGACCCGGTCTCTTCAGTAAAAGTGGACAACGGTCTGATCGTCGAGCTGGAC GGCAAACGCCGGGACCAGTTTGACATGATCGACCGATTTATCGCCGATTACGC GATCAACGTTGAGCGCACAGAGCAGGCAATGCGCCTGGAGGCGGTGGAAATAG CCCGCATGCTGGTGGATATTCACGTCAGTCGGGAGGAGATCATTGCCATCACT ACCGCCATCACGCCGGCCAAAGCGGTCGAGGTGATGGCGCAGATGAACGTGGT GGAGATGATGATGGCGCTGCAGAAGATGCGTGCCCGCCGGACCCCCTCCAACC AGTGCCACGTCACCAATCTCAAAGATAATCCGGTGCAGATTGCTGCTGACGCC GCCGAGGCCGGGATCCGCGGCTTCTCAGAACAGGAGACCACGGTCGGTATC**AT** GCGCTATGCGCCGTTTAACGCCCTGGCGCTGTTGGTCGGTTCGCAGTGCGGCC GCCCCGGCGTTTTGACGCAGTGCTCGGTGGAAGAGGCCACCGAGCTGGAGCTG GGCATGCGTGGCTTAACCAGCTACGCCGAGACGGTGTCGGTCTACGGCACCGA AGCGGTATTTACCGACGGCGATGATACTCCGTGGTCAAAGGCGTTCCTCGCCT CGGCCTACGCCTCCCGCGGGTTGAAAATGCGCTACACCTCCGGCACCGGATCC GAAGCGCTGATGGGCTATTCGGAGAGCAAGTCGATGCTCTACCTCGAATCGCG CTGCATCTTCATTACCAAAGGCGCCGGGGTTCAGGGGCTGCAAAACGGCGCGG TGAGCTGTATCGGCATGACCGGCGCTGTGCCGTCGGGCATTCGGGCGGTGCTG GCGGAAAACCTGATCGCCTCTATGCTCGACCTCGAAGTGGCGTCCGCCAACGA CCAGACTTTCTCCCACTCGGATATTCGCCGCACCGCGCGCACCCTGATGCAGA TGCTGCCGGGCACCGACTTTATTTTCTCCGGCTACAGCGCGGTGCCGAACTAC GACAACATGTTCGCCGGCTCGAACTTCGATGCGGAAGATTTTGATGATTACAA CATCCTGCAGCGTGACCTGATGGTTGACGGCGGCCTGCGTCCGGTGACCGAGG CGGAAACCATTGCCATTCGCCAGAAAGCGGCGCGGGCGATCCAGGCGGTTTTC CGCGAGCTGGGGCTGCCGCCAATCGCCGACGAGGAGGTGGAGGCCGCCACCTA CGCGCACGGTAGCAACGAGATGCCGCCGCGTAACGTGGTGGAGGATCTGAGTG CGGTGGAAGAGATGATGAAGCGCAACATCACCGGCCTCGATATTGTCGGCGCG CTGAGCCGCAGCGGCTTTGAGGATATCGCCAGCAATATTCTCAATATGCTGCG CCAGCGGGTCACCGGCGATTACCTGCAGACCTCGGCCATTCTCGATCGGCAGT TCGAGGTGGTGAGTGCGGTCAACGACATCAATGACTATCAGGGGCCGGGCACC GGCTATCGCATCTCTGCCGAACGCTGGGCGGAGATCAAAAATATTCCGGGCGT GGTTCAGCCCGACACCATTGAA**C**AAGGCGGTATTCCTGTGCAACAGACAACCC AAATTCAGCCCTCTTTTACCCTGAAAACCCGCGAGGGCGGGGTAGCTTCTGCC GATGAACGCGCCGATGAAGTGGTGATCGGCGTCGGCCCTGCCTTCGATAAACA CCAGCATCACACTCTGATCGATATGCCCCATGGCGCGATCCTCAAAGAGCTGA TTGCCGGGGTGGAAGAAGAGGGGCTTCACGCCCGGGTGGTGCGCATTCTGCGC ACGTCCGACGTCTCCTTTATGGCCTGGGATGCGGCCAACCTGAGCGGCTCGGG GATCGGCATCGGTATCCAGTCGAAGGGGACCACGGTCATCCATCAGCGCGATC TG**TG**GCCGCTCAGCAACCTGGAGCTGTTCTCCCAGGCGCCGCTGCTGACGCTG GAAACCTACCGGCAGATTGGCAAAAACGCCGCGCGCTATGCGCGCAAAGAGTC ACCTTCGCCGGTGCCGGTGGTGAACGATCAG**TGG**GTGCGGCCGAAATTTATGG CCAAAGCCGCGCTATTTCATATCAAAGAGACCAAACATGTGGTGCAGGACGCC GAGCCCGTCACCCTGCACGTCGACTTAGTAAGGGAGTGA  (SEQ  ID  NO.: 24) |

Example 3-4. Production of 3-HP through flask culture of recombinant *E. coli* containing variants

In consideration of the oxygen resistance and high enzymatic reaction rate, the variants of the present invention were expected to produce sufficient 3-HP even under conditions containing low concentrations of expensive coenzyme B12, and this was verified.

Specifically, a plasmid co-expressing the seven genes of the variants of Example 3-4 and glycerol dehydratase reactivase (GDHt reactivase) was constructed. The constructed plasmid was introduced into *E. coli* W strains along with a plasmid expressing α-ketoglutaric semialdehyde dehydrogenase (KGSADH), resulting in recombinant *E. coli* strains that were cultured to produce 3-HP (for detailed methods, see Park, Y.S., Choi, U.J., Nam, N.H. et al., Sci Rep. 7, 17155 (2017)).

The recombinant cells were cultured in modified M9 (2 g/L NaCl, 1 g/L yeast extract, 2 g/L NH$_4$Cl, 0.5 g/L MgSO$_4$ · 7H$_2$O, and 100 mM potassium phosphate buffer, pH 7.0) and 100 mM glycerol. To stabilize the plasmid, 34 μg/mL of chloramphenicol and 200 μg/mL of ampicillin were added to the medium. A single colony was cultured in a 4 ml test tube at 37°C and 180 rpm for one day, and then 20 ml of the culture was transferred to a 100 ml flask such that OD$_{600}$ reached 0.1, and cultured under the same temperature and rotation conditions until the OD$_{600}$ became 1.0. 50 ml of the culture was transferred to a 250 ml flask and cultured, and it was transferred such that OD$_{600}$ became 0.1, and cultured at 37°C and 180 rpm in a light-blocking state until the OD$_{600}$ became between 0.9 and 1.0. Protein expression was then induced by adding 0.1 mM IPTG and various concentrations of coenzyme B12 (2.0, 0.4, 0.08, and 0.016 μM). The cells were cultured for 24 hours. The pH was measured at 8 and 16 hours, and the pH was adjusted to 7.0 with 10 M NaOH. The culture medium was harvested and centrifuged at 13,000 rpm for 10 minutes, and the 3HP concentration was analyzed from the supernatant using HPLC.

The result of the experiment showed that, in the presence of 0.016 μM coenzyme B12, the 3HP production was increased by 0.95 times (L113F(β)), 1.07 times (L113W(β)), 2.33 times (M158W(β)), 2.69 times (A177M(α)), 1.47 times (A177M/L113W(α/β)), 4.07 times (A177M/M158W(α/β)), 1.86 times (A177M/L113W/M158W(α/β/β)) compared to fGDHt, and in the presence of 0.08 μM coenzyme B12, the 3HP production was increased by 1.12 times (L113F(β)), 1.21 times (L113W(β)), (1.36 times M158W(β)), 1.56 times (M158W(β)), 1.26 times (A177M(α)), 1.8 times (A177M/M158W(α/β)), and 1.34 times (A177M/L113W/M158W(α/β/β)).

In particular, the A177M(α) and A177M/M158W (α/β) variants exhibited higher 3HP production compared to fGDHt across all time points (A177M(α): 2.69 times 0.016 μM, 1.56 times 0.08 μM, 1.12 times 0.4 μM, 1.23 times 2.0 μM; A177M/M158W (α/β): 4.07 times 0.016 μM, 1.8 times 0.08 μM, 1.23 times 0.4 μM, 1.25 times 2.0 μM).

## Industrial Applicability

The fused glycerol dehydratase variant according to the present invention exhibits high activity to glycerol as well as excellent resistance to oxygen, thereby solving the problem of inactivation of coenzyme B12 by oxygen. Furthermore, the recombinant strain containing the variant exhibits high activity even in media containing low concentrations of coenzyme B12, thereby reducing costs associated with coenzyme B12.

Although specific configurations of the present invention have been described in detail, those skilled in the art will appreciate that this detailed description is provided as preferred embodiments for illustrative purposes, and should not be construed as limiting the scope of the present invention. Therefore, the substantial scope of the present invention is defined by the accompanying filed claims and equivalents thereto.

## Sequence Listing Free Text

An electronic file is attached.

## Claims

1. A recombinant glycerol dehydratase variant comprising a fusion of an alpha subunit and a beta subunit of glycerol dehydratase (GDHt),
   wherein the recombinant glycerol dehydratase variant comprises a substitution of at least one amino acid residue selected from the group consisting of:

   G19, L29, A177, A207, Y271, and V455 of the alpha subunit represented by SEQ ID NO: 1; and
   L113, S149, and M158 of the beta subunit represented by SEQ ID NO: 2.

2. The recombinant glycerol dehydratase variant according to claim 1, wherein the variant comprises at least one substitution selected from the group consisting of G19A(α), L29F(α), A177M(α), A207W(α), Y271W(α), and V455W(α).

3. The recombinant glycerol dehydratase variant according to claim 1, wherein the variant comprises at least one substitution selected from the group consisting of L113Y(β), L113F(β), L113W(β), S149I(β), S149M(β), M158W(β), and M158Y(β).

4. The recombinant glycerol dehydratase variant according to claim 1, wherein the variant comprises at least one substitution selected from the group consisting of:

A177M($\alpha$), L113W($\beta$), and M158W($\beta$);
A177M($\alpha$) and L113W($\beta$); and
A177M($\alpha$) and M158W($\beta$).

5. The recombinant glycerol dehydratase variant according to claim 1, wherein the alpha subunit and the beta subunit are linked via a linker.

6. The recombinant glycerol dehydratase variant according to claim 5, wherein the linker comprises any one selected from the group consisting of:

$(G_4S)_a$, $G(PT)_3T(PT)_cG$, and QGGIPV,
wherein a is an integer from 1 to 8, and b and c are integers from 2 to 10.

7. The recombinant glycerol dehydratase variant according to claim 5, wherein the linker is disposed between a C-terminus of the alpha subunit and an N-terminus of the beta subunit.

8. A nucleic acid encoding the fused glycerol dehydratase variant according to claim 1.

9. A recombinant microorganism comprising the nucleic acid according to claim 8.

10. A recombinant microorganism transformed with the nucleic acid according to claim 8 or the recombinant vector according to claim 9.

11. The recombinant microorganism according to claim 10, further expressing an aldehyde dehydrogenase.

12. The recombinant microorganism according to claim 10, further expressing a 1,3-propanediol oxidoreductase.

13. A method of producing 3-hydroxypropionic acid, the method comprising:

culturing the recombinant microorganism according to claim 11 in a medium containing glycerol to produce 3-hydroxypropionic acid; and
recovering the produced 3-hydroxypropionic acid.

14. A method of producing 1,3-propanediol, the method comprising:

culturing the recombinant microorganism according to claim 12 in a medium containing glycerol to produce 1,3-propanediol; and
recovering the produced 1,3-propanediol.

15. A pharmaceutical composition for preventing or treating cancer for co-administration with a PD-1 antagonist or a PD-L1 antagonist, the pharmaceutical composition comprising:
a fusion protein comprising VEGFR1 (vascular endothelial growth factor receptor 1) domain 2, VEGFR1 domain 3, and an Fc region of an antibody as an active ingredient.

[FIG.1]

[FIG.2]

[FIG.3]

[FIG.4]

[FIG.5]

(a)

[FIG.6]

A

B

$(Gly_4Ser)_4$

$Gly(ProThr)_4Thr(ProThr)_7Gly$

$GlnGlyGlyIleProThr$

[FIG.7]

Results of enzyme purification
based on α-β subunit linkage

Resistance to oxygen-induced
inactivation

| | Fused W.T | Non-Fused W.T |
|---|---|---|
| HalfLife | 3.268 | 2.388 |

Enzyme activity to
substrates

| | $K_m$ (mM) | $K_{cat}$ ($s^{-1}$) | $K_{cat}/K_m$ ($s^{-1}$ $mM^{-1}$) |
|---|---|---|---|
| WT(non-fused) | 0.37± 0.03 | 63.77 ± 2.73 | $1.7 \times 10^2$ |
| fGDHt | 0.27± 0.03 | 230.28 ± 10.63 | $8.5 \times 10^2$ |

[FIG.8]

[FIG.9]

[FIG.10]

[FIG.11]

5min Oxygen Inactivation Test Relative Value (%)

[FIG.12]

[FIG.13]

EP 4 722 358 A1

# INTERNATIONAL SEARCH REPORT

| International application No. |
|---|
| **PCT/KR2024/007479** |

| A. | CLASSIFICATION OF SUBJECT MATTER |
|---|---|

**C12N 9/88**(2006.01)i; **C12P 7/52**(2006.01)i

According to International Patent Classification (IPC) or to both national classification and IPC

| B. | FIELDS SEARCHED |
|---|---|

Minimum documentation searched (classification system followed by classification symbols)

C12N 9/88(2006.01); A61K 31/47(2006.01); A61K 39/395(2006.01); C07K 14/195(2006.01); C12N 1/21(2006.01); C12N 15/52(2006.01); C12Q 1/527(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 글리세롤 탈수소효소(glycerol dehydrogenase), 서브유닛(subunit), 재조합 (recombination), 서열(sequence), 변이체(variant), 링커(linker), 핵산(nucleic acid), 벡터(vector), 숙주세포(host cell), 혈관내피 성장인자 수용체(vascular endothelial growth factor receptor 1,VEGFR1), 융합단백질(fusion protein)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2017-0122809 A (MERCK SHARP & DOHME CORP. et al.) 06 November 2017 (2017-11-06) See abstract; claims 1-6; and paragraphs [0059]-[0061], [0131], [0134], [0140] and [0173]. | 15 |
| A | | 1-14 |
| X | KR 10-2022-0149740 A (MERCK SHARP & DOHME LLC et al.) 08 November 2022 (2022-11-08) See abstract; claims 1 and 17-20; and paragraphs [0057]-[0059], [0156] and [0206]. | 15 |
| A | KR 10-2014-0032057 A (SAMSUNG ELECTRONICS CO., LTD.) 14 March 2014 (2014-03-14) See abstract; claims 1, 2, 7, 8 and 10-12; paragraph [0036]; and pages 20-24. | 1-15 |
| A | KR 10-2014-0135803 A (E.I. DU PONT DE NEMOURS AND COMPANY) 26 November 2014 (2014-11-26) See paragraphs [0065], [0066] and [0213]; and pages 53-55. | 1-15 |

| ✓ Further documents are listed in the continuation of Box C. | ✓ See patent family annex. |
|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **09 September 2024** | **09 September 2024** |

| Name and mailing address of the ISA/KR | Authorized officer |
|---|---|
| **Korean Intellectual Property Office Government Complex-Daejeon Building 4, 189 Cheongsa- ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2024/007479** |

### C.    DOCUMENTS CONSIDERED TO BE RELEVANT

| Category* | Citation of document, with indication, where appropriate, of the relevant passages | Relevant to claim No. |
|---|---|---|
| A | NCBI Reference Sequence: WP_002917676.1. MULTISPECIES: propanediol/glycerol family dehydratase large subunit [Enterobacterales]. (22 May 2021).<br>    See page 1. | 1-15 |
| A | US 7410754 B1 (GIBSON, K. J. et al.) 12 August 2008 (2008-08-12)<br>    See entire document. | 1-15 |

**INTERNATIONAL SEARCH REPORT**

| International application No. |
| --- |
| **PCT/KR2024/007479** |

| Box No. I          Nucleotide and/or amino acid sequence(s) (Continuation of item 1.c of the first sheet) |
| --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/KR2024/007479**

| Patent document cited in search report | | | Publication date (day/month/year) | Patent family member(s) | | | Publication date (day/month/year) |
|---|---|---|---|---|---|---|---|
| KR | 10-2017-0122809 | A | 06 November 2017 | JP | 2016-196411 | A | 24 November 2016 |
| | | | | JP | 2018-512391 | A | 17 May 2018 |
| | | | | JP | 2021-035962 | A | 04 March 2021 |
| | | | | JP | 2023-039448 | A | 20 March 2023 |
| | | | | JP | 6788600 | B2 | 25 November 2020 |
| | | | | JP | 7507209 | B2 | 27 June 2024 |
| | | | | KR | 10-2024-0064733 | A | 13 May 2024 |
| | | | | KR | 10-2662228 | B1 | 02 May 2024 |
| | | | | US | 11147806 | B2 | 19 October 2021 |
| | | | | US | 11547705 | B2 | 10 January 2023 |
| | | | | US | 2018-0092901 | A1 | 05 April 2018 |
| | | | | US | 2022-0023285 | A1 | 27 January 2022 |
| | | | | WO | 2016-140717 | A1 | 09 September 2016 |
| | | | | WO | 2016-141218 | A1 | 09 September 2016 |
| KR | 10-2022-0149740 | A | 08 November 2022 | CN | 115443152 | A | 06 December 2022 |
| | | | | EP | 4114464 | A1 | 11 January 2023 |
| | | | | EP | 4114464 | A4 | 01 May 2024 |
| | | | | JP | 2023-515675 | A | 13 April 2023 |
| | | | | US | 2023-0118596 | A1 | 20 April 2023 |
| | | | | WO | 2021-178657 | A1 | 10 September 2021 |
| KR | 10-2014-0032057 | A | 14 March 2014 | None | | | |
| KR | 10-2014-0135803 | A | 26 November 2014 | CN | 104245723 | A | 24 December 2014 |
| | | | | EP | 2822959 | A1 | 14 January 2015 |
| | | | | JP | 2015-509378 | A | 30 March 2015 |
| | | | | US | 2013-0230892 | A1 | 05 September 2013 |
| | | | | US | 2014-0147899 | A1 | 29 May 2014 |
| | | | | US | 2014-0155578 | A1 | 05 June 2014 |
| | | | | US | 8686114 | B2 | 01 April 2014 |
| | | | | WO | 2013-134167 | A1 | 12 September 2013 |
| US | 7410754 | B1 | 12 August 2008 | CN | 102031265 | A | 27 April 2011 |
| | | | | CN | 1965088 | A | 16 May 2007 |
| | | | | EP | 1583818 | A2 | 12 October 2005 |
| | | | | EP | 1583818 | A4 | 15 August 2007 |
| | | | | JP | 2007-524342 | A | 30 August 2007 |
| | | | | KR | 10-2005-0089970 | A | 09 September 2005 |
| | | | | US | 2008-0293119 | A1 | 27 November 2008 |
| | | | | US | 2011-0021767 | A1 | 27 January 2011 |
| | | | | US | 2011-0021768 | A1 | 27 January 2011 |
| | | | | US | 7985849 | B2 | 26 July 2011 |
| | | | | US | 8445659 | B2 | 21 May 2013 |
| | | | | US | 8569469 | B2 | 29 October 2013 |
| | | | | WO | 2004-056963 | A2 | 08 July 2004 |
| | | | | WO | 2004-056963 | A3 | 08 September 2006 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 85694692013 A, Gibson
- US 8569469 B, Gibson,

- US 2013 A

**Non-patent literature cited in the description**

- **DROZDZYNSKA, A. et al.** *BioTechnologia*, 2011, vol. 92 (1), 92-100
- **VALDEHUESA et al.** *Applied microbiology and biotechnology*, 2013, vol. 97, 3309-3321
- **MITREA, L. et al.** *Microbial Cell Factories*, 2017, vol. 16, 1-17
- **TORAYA, T. et al.** *Biochemistry*, 1979, vol. 18 (3), 417-426
- **TORAYA, T.** ; **ABELES, R. H.** *Archives of Biochemistry and Biophysics*, 1980, vol. 203 (1), 174-180
- **MANCIA et al.** *Structure*, 1996, vol. 4 (3), 339-350
- **SHIBATA, N. et al.** *Structure*, 1999, vol. 7 (8), 997-1008
- **KAMACHI, T. et al.** *Chemistry-A European Journal*, 2007, vol. 13 (28), 7864-7873
- **POZNANSKAJA, A. et al.** *Archives of Biochemistry and Biophysics*, 1979, vol. 194 (2), 379-386
- **YAMANISHI, M. et al.** *European journal of biochemistry*, 2002, vol. 269 (18), 4484-4494
- **DOITOMI, K. et al.** *Bulletin of the Chemical Society of Japan*, 2014, vol. 87 (9), 950-959
- **BILIC, L. et al.** *The Journal of Physical Chemistry B*, 2019, vol. 123 (29), 6178-6187
- **TORAYA, T.** *Cellular and Molecular Life Sciences CMLS*, 2000, vol. 57, 106-127
- **LIAO, D. I. et al.** *Structure*, 2003, vol. 11 (1), 109-119
- **SHIBATA, N. et al.** *Structure*, 2005, vol. 13 (12), 1745-1754
- **TOBIMATSU, T. et al.** *Archives of microbiology*, 2000, vol. 174, 81-88
- **YAMANISHI, M. et al.** *The FEBS journal*, 2012, vol. 279 (5), 793-804
- **SHIBATA, N. et al.** *Angewandte Chemie*, 2018, vol. 130 (26), 7956-7961
- **LIAO, D. I. et al.** *Journal of inorganic biochemistry*, 2003, vol. 93 (1-2), 84-91

- **WANG, X. D. et al.** *Biotechnol Lett*, 2009, vol. 31 (5), 711-717
- **MADDOCK, D. J. et al.** *Biotechnol J*, 2017, vol. 12 (12)
- **MITREA L et al.** *Microbial Cell Factories*, 2017, vol. 16, 1-17
- **TORAYA T et al.** *Archives of Biochemistry and Biophysics*, 1980, vol. 203 (1), 174-180
- **SHIBATA N et al.** *Structure*, 1999, vol. 7 (8), 997-1008
- **KAMACHI T et al.** *Chemistry-A European Journal*, 2007, vol. 13 (28), 7864-7873
- **POZNANSKAJA A et al.** *Archives of Biochemistry and Biophysics*, 1979, vol. 194 (2), 379-386
- **YAMANISHI M et al.** *European journal of biochemistry*, 2002, vol. 269 (18), 4484-4494
- **DOITOMI K et al.** *Bulletin of the Chemical Society of Japan*, 2014, vol. 87 (9), 950-959
- **TORAYA T.** *Cellular and Molecular Life Sciences CMLS*, 2000, vol. 57, 106-127
- **LIAO D I et al.** *Structure*, 2003, vol. 11 (1), 109-119
- **SHIBATA N et al.** *Structure*, 2005, vol. 13 (12), 1745-1754
- **TOBIMATSU T et al.** *Archives of microbiology*, 2000, vol. 174, 81-88
- **YAMANISHI M et al.** *The FEBS journal*, 2012, vol. 279 (5), 793-804
- **SHIBATA N et al.** *Angewandte Chemie*, 2018, vol. 130 (26), 7956-7961
- **LIAO D I et al.** *Journal of inorganic biochemistry*, 2003, vol. 93 (1-2), 84-91
- **WANG X D et al.** *Biotechnol Lett*, 2009, vol. 31 (5), 711-717
- **MADDOCK D J et al.** *Biotechnol J*, 2017, vol. 12 (12)
- **R.K. SAXENA et al.** *Biotechnology Advances*, 2009, vol. 27 (6), 895-913
- **PARK, Y.S.** ; **CHOI, U.J.** ; **NAM, N.H. et al.** *Sci Rep.*, 2017, vol. 7, 17155